# EUROPEAN PATENT APPLICATION

(11) **EP 2 762 136 A1**
(43) Date of publication of application: **06.08.2014**
(21) Application number: 14001452.3
(22) Date of filing: 29.01.2007
(51) Int. Cl.: A61K 31/365, A61P 25/28

(54) **Methods of stimulating cellular growth, synaptic remodeling and consolidation of long-term memory**

(30) Priority: 28.07.2006 US 833785 P
(62) Divisional of application: 07749484.7
(71) Applicant: Blanchette Rockefeller Neurosciences Institute, Morgantown, WV 26505-3409 (US)
(72) Inventor: Alkon, Daniel L., Bethesda, Maryland 20817 (US); Hongpaisan, Jarin, Rockville, Maryland 20850 (US)
(74) Representative: Kador & Partner

(57) **Abstract**

The present invention provides methods of slowing or reversing the loss of memory and learning comprising the steps of contacting an effective amount of a PKC activator with a protein kinase C (PKC) in a subject identified with memory loss slowing or reversing memory loss. The present invention provides methods of stimulating cellular growth, neuronal growth, dendritic growth, dendritic spine formation, dendritic spine density, and the translocation of ELAV to proximal dendrites, and synaptic remodeling. The present invention also provides methods of contacting a protein kinase C (PKC) activator with a PKC activator in a manner sufficient to stimulate the synthesis of proteins sufficient to consolidate long-term memory. The present invention also provides methods of contacting a protein kinase C (PKC) activator with a PKC activator in a manner sufficient to downregulate PKC.

## Description

### FIELD OF THE INVENTION

The present invention relates to methods of upregulating and downregulating protein kinase C that are useful for stimulating cellular growth, synaptic remodeling and enhancing memory and the treatment of cell proliferative disorders.

### BACKGROUND OF THE INVENTION

Various disorders and diseases exist which affect cognition. Cognition can be generally described as including at least three different components: attention, learning, and memory. Each of these components and their respective levels affect the overall level of a subject's cognitive ability. For instance, while Alzheimer's Disease patients suffer from a loss of overall cognition and thus deterioration of each of these characteristics, it is the loss of memory that is most often associated with the disease. In other diseases patients suffer from cognitive impairment that is more predominately associated with different characteristics of cognition. For instance, Attention Deficit Hyperactivity Disorder (ADHD), focuses on the individual's ability to maintain an attentive state. Other conditions include general dementias associated with other neurological diseases, aging, and treatment of conditions that can cause deleterious effects on mental capacity, such as cancer treatments, stroke/ischemia, and mental retardation.

The requirement of protein synthesis for long-term memory has been demonstrated over several decades for a variety of memory paradigms. Agranoff et al. (1967) Science 158: 1600-1601; Bergold et al. (1990) Proc. Natl. Acad. Sci. 87:3788-3791; Cavallaro et al. (2002) Proc. Natl. Acad. Sci. 99: 13279-16284; Crow et al. (1990) Proc. Natl. Acad. Sci. 87: 4490-4494; Crow et al. (1999) J. Neurophysiol. 82: 495-500; Epstein et al. (2003) Neurobiol. Learn. Mem. 79: 127-131; Ezzeddine et al. (2003) J. Neurosci. 23: 9585-9594; Farley et al. (1991) Proc. Natl. Acad. Sci. 88: 2016-2020; Flexner et al. (1996) Proc. Natl. Acad. Sci. 55: 369-374; Hyden et al. (1970) Proc. Natl. Acad. Sci. 65: 898-904; Nelson et al. (1990) Proc. Natl. Acad. Sci. 87: 269-273; Quattrone et al. (2001) Proc. Natl. Acad. Sci. 98: 11668-11673; Zhao et al. (1999) J. Biol. Chem. 274: 34893-34902; Zhao et al. (2000) FASEB J. 14: 290-300. Flexner originally showed that drug-induced inhibition of protein synthesis (*e.g*., with 5-propyluracil or anisomycin) blocked long-term memory when this inhibition occurred during a critical time interval following the training paradigm. Flexner et al. (1996) Proc. Natl. Acad. Sci. 55: 369-374. If protein synthesis was inhibited *before* this critical time window or at any time *after* this window, there was no effect on long-term memory. The identity of the proteins essential for memory consolidation, the mechanisms of their regulation, and their role in the consolidation of long-term memory has remained a mystery.

In many species the formation of long-term associative memory has also been shown to depend on translocation, and thus activation, of protein kinase C (PKC) isozymes to neuronal membranes. Initially, these PKC isozymes, when activated by a combination of calcium and co-factors, such as diacylglycerol, achieve a stable association with the inner aspect of the external neuronal membrane and membranes of internal organelle, such as the endoplasmic reticulum. PKC activation has been shown to occur in single identified Type B cells of the mollusk *Hermissenda* (McPhie et al. (1993) J. Neurochem. 60: 646-651), a variety of mammalian associative learning protocols, including rabbit nictitating membrane conditioning (Bank et al. (1988) Proc. Natl. Acad. Sci. 85: 1988-1992; Olds et al. (1989) Science 245: 866-869), rat spatial maze learning (Olds et al. (1990) J. Neurosci. 10: 3707-3713), and rat olfactory discrimination learning, upon Pavlovian conditioning. Furthermore, calexcitin (Nelson et al. (1990) Science 247: 1479-1483), a high-affinity substrate of the alpha isozyme of PKC increased in amount and phosphorylation (Kuzirian et al. (2001) J. Neurocytol. 30: 993-1008) within single identified Type B cells in a Pavlovian-conditioning-dependent manner.

There is increasing evidence that the individual PKC isozymes play different, sometimes opposing, roles in biological processes, providing two directions for pharmacological exploitation. One is the design of specific (preferably, isozyme specific) inhibitors of PKC. This approach is complicated by the fact that the catalytic domain is not the domain primarily responsible for the isotype specificity of PKC. The other approach is to develop isozyme-selective, regulatory site-directed PKC activators. These may provide a way to override the effect of other signal transduction pathways with opposite biological effects. Alternatively, by inducing down-regulation of PKC after acute activation, PKC activators may cause long term antagonism.

Following associative memory protocols, increased PKC association with the membrane fractions in specific brain regions can persist for many days (Olds et al. (1989) Science 245: 866-869). Consistent with these findings, administration of the potent PKC activator bryostatin, enhanced rats spatial maze learning (Sun et al. (2005) Eur. J. Pharmacol. 512: 45-51). Furthermore, clinical trials with the PKC activator, bryostatin, suggested (Marshall et al. (2002) Cancer Biology & Therapy 1: 409-416) that PKC activation effects might be enhanced by an intermittent schedule of drug delivery. One PKC activator, bryostatin, a macrolide lactone, activates PKC in sub-nanomolar concentrations (Talk et al. (1999) Neurobiol. Learn. Mem. 72: 95-117). Like phorbol esters and the endogenous activator DAG, bryostatin binds to the C1 domain within PKC and causes its translocation to membranes, which is then followed by downregulation.

The non-tumorigenic PKC activator, bryostatin, has undergone extensive testing in humans for the treatment of cancer in doses (25 µ g/m²-120 µ g/m²) known to cause initial PKC activation followed by prolonged downregulation (Prevostel et al. (2000) Journal of Cell Science 113: 2575-2584; Lu et al. (1998) Mol. Biol. Cell 18: 839-845; Leontieva et al. (2004) J. Biol. Chem. 279:5788-5801). Bryostatin activation of PKC has also recently been shown to activate the alpha-secretase that cleaves the amyloid precursor protein (APP) to generate the non-toxic fragments soluble precursor protein (sAPP) from human fibroblasts (Etcheberrigaray et al. (2004) Proc. Natl. Acad. Sci. 101: 11141-11146). Bryostatin also enhances learning and memory retention of the rat spatial maze task (Sun et al. (2005) Eur. J. Pharmacol. 512: 45-51), learning of the rabbit nictitating membrane paradigm (Schreurs and Alkon, unpublished), and in a preliminary report, *Hermissenda* conditioning (Scioletti et al. (2004) Biol. Bull. 207: 159). Accordingly, optimal activation of PKC is important for many molecular mechanisms that effect cognition in normal and diseased states.

Because the upregulation of PKC is difficult to achieve without downregulation, and vice versa, methods of upregulation of PKC while minimizing downregulation are needed to enhance the cognitive benefits observed associated with PKC activation. The methods and compositions of the present invention fulfill these needs and will greatly improve the clinical treatment for Alzheimer's disease and other neurodegenerative diseases, as well as, provide for improved cognitive enhancement prophylactically. The methods and compositions also provide treatment and/or enhancement of the cognitive state through the modulation of α-secretase.

### SUMMARY OF THE INVENTION

This invention relates to a method of contacting a PKC activator with protein kinase C in a manner sufficient to stimulate the synthesis of proteins sufficient to consolidate long term memory.

This invention relates to a method comprising the step of contacting a PKC activator with a protein kinase C (PKC) to stimulate cellular growth.

This invention relates to a method comprising the step of contacting a PKC activator with a protein kinase C (PKC) to stimulate neuronal growth.

This invention relates to a method comprising the step of contacting a PKC activator with a protein kinase C (PKC) to stimulate dendritic growth.

This invention relates to a method comprising the step of contacting a PKC activator with a protein kinase C (PKC) to stimulate dendritic spine formation.

This invention relates to a method comprising the step of contacting a PKC activator with a protein kinase C (PKC) to stimulate dendritic spine density.

This invention relates to a method comprising the step of contacting a PKC activator with a protein kinase C (PKC) to stimulate ELAV translocation to proximal dendrites.

The present invention provides methods of slowing or reversing the loss of memory and learning comprising the steps of contacting an effective amount of a PKC activator with a protein kinase C (PKC) in a subject identified with memory loss slowing or reversing memory loss. In one embodiment, the contacting of an effective amount of a PKC activator with ah PKC stimulates cellular or neuronal growth. In another embodiment, the contacting of an effective amount of a PKC activator with a PKC stimulates dendritic growth. In yet another embodiment, the contacting of an effective amount of a PKC activator with ah PKC stimulates dendritic spine formation. In yet another embodiment, the contacting of an effective amount of a PKC activator with ah PKC stimulates dendritic spine density.

The present invention also provides methods of stimulating cellular or neuronal growth comprising the steps of contacting a effective amount of a PKC activator with a protein kinase C (PKC) in a subject, thereby stimulating cellular or neuronal growth. In one embodiment, the subject is identified as having impaired learning or memory. In another embodiment, the contacting of an effective amount of a PKC activator with a PKC stimulates dendritic growth. In yet another embodiment, the contacting of an effective amount of a PKC activator with ah PKC stimulates dendritic spine formation. In yet another embodiment, the contacting of an effective amount of a PKC activator with ah PKC stimulates dendritic spine density.

In one embodiment, the PKC activator is a macrocyclic lactone. In one embodiment, the PKC activator is a benzolactam. In one embodiment, the PKC activator is a pyrrolidinone. In a preferred embodiment, the macrocyclic lactone is bryostatin. In a more preferred embodiment, the bryostatin is bryostatin-1, -2, -3, -4, -5, -6, -7, -8, -9, -10, -11, -12, -13, -14, -15, -16, -17, or -18. In the most preferred embodiment, the bryostatin is bryostatin-1.

In one embodiment, the macrocyclic lactone is neristatin. In a preferred embodiment, the neristatin is neristatin-1.

In one embodiment, the contact activates PKC. In one embodiment, the contact increases the amount of PKC. In one embodiment, the contact increases the synthesis of PKC. In one embodiment, the contact increases the amount of calexcitin. In one embodiment, the contact does not result in substantial subsequent deregulation of PKC.

In one embodiment, the contact is repeated. In another embodiment, the contact is repeated at regular intervals. In another embodiment, the interval is between one week to one month, one day and one week, or less than one hour and 24 hours. In another embodiment, the interval is between one week and one month. In another embodiment, the interval is between one day and one week. In another embodiment, the interval is between less than one hour and 24 hours.

In one embodiment, the contact is maintained for a fixed duration. In another embodiment, the fixed duration is less than 24 hours. In another embodiment, the fixed duration is less than 12 hours. In another embodiment, the fixed duration is less than 6 hours. In another embodiment, the fixed duration is less than 6 hours. In another embodiment, the fixed duration is less than 4 hours. In another embodiment, the fixed duration is less than 2 hours. In a preferred embodiment, the fixed duration is between about 1 and 12 hours. In a more preferred embodiment, the fixed duration is between about 2 and 6 hours. In the most preferred embodiment, the fixed duration is about 4 hours.

In one embodiment, the contact is repeated for a period greater than one day. In another embodiment, the contact is repeated for a period between one day and one month. In another embodiment, the contact is repeated for a period between one day and one week. In another embodiment, the contact is repeated for a period between one week and one month. In another embodiment, the contact is repeated for a period between one month and six months. In another embodiment, the contact is repeated for a period of one month. In another embodiment, the contact is repeated for a period greater than one month.

In one embodiment, the PKC activator is administered in a manner that enhances delivery to the brain or central nervous system and minimizes delivery to peripheral tissues. In another embodiment, the PKC activator is administered in a manner that enhances transport of the PKC activator across the blood-brain barrier. In one embodiment, PKC activators are formulated in a pharmaceutical composition that enhances delivery to the brain or central nervous system and minimizes delivery to peripheral tissues. In another embodiment, the PKC activators of the present invention are administered in an artificial LDL particle as disclosed in U.S. Publication No. 20040204354, which is incorporated herein by reference in its entirety. In another embodiment, the PKC activator is formulated in an artificial LDL particle. In yet another embodiment, the PKC activator is conjugated to cholesterol and formulated in an artificial LDL particle.

The invention relates to a method of contacting a PKC activator with protein kinase C in a manner sufficient to downregulate PKC.

In one embodiment, the PKC activator is a macrocyclic lactone. In one embodiment, the PKC activator is a benzolactam. In one embodiment, the PKC activator is a pyrrolidinone. In a preferred embodiment, the macrocyclic lactone is bryostatin. In a more preferred embodiment, the bryostatin is bryostatin-1, -2, -3, -4, -5, -6, -7, -8, -9, -10, -11, -12, -13, -14, -15, -16, -17, or -18. In the most preferred embodiment, the bryostatin is bryostatin-1.

In one embodiment, the macrocyclic lactone is neristatin. In a preferred embodiment, the neristatin is neristatin-1.

In one embodiment, the contact does not stimulate the synthesis of PKC. In another embodiment, the contact does not substantially stimulate the synthesis of PKC. In another embodiment, the contact decreases the amount of PKC. In another embodiment, the contact substantially decreases the amount of PKC. In another embodiment, the contact does not stimulate the synthesis of calexcitin.

In one embodiment, the contact is for a sustained period. In one embodiment, the sustained period if between less than one hour and 24 hours. In another embodiment, the sustained period is between one day and one week. In another embodiment, the sustained period is between one week and one month. In another embodiment, the sustained period is between less than one hour and 12 hours. In another embodiment, the sustained period is between less than one hour and 8 hours. In another embodiment, the sustained period is between less than one hour and 4 hours. In a preferred embodiment, the sustained period is about 4 hours.

In one embodiment, the contact produces sustained downregulation of PKC. In one embodiment, the sustained period if between less than one hour and 24 hours. In another embodiment, the sustained period is between one day and one week. In another embodiment, the sustained period is between one week and one month. In another embodiment, the sustained period is between less than one hour and 12 hours. In another embodiment, the sustained period is between less than one hour and 8 hours. In another embodiment, the sustained period is between less than one hour and 4 hours. In a preferred embodiment, the sustained period is about 4 hours.

This invention relates to a method of contacting a PKC activator with protein kinase C in a manner sufficient to stimulate the synthesis of proteins sufficient to consolidate long term memory, further comprising the step of inhibiting degradation of PKC.

In one embodiment, the degradation is through ubiquitination. In another embodiment, the degradation is inhibited by lactacysteine. In another embodiment, the PKC is human.

This invention relates to a method of contacting a PKC activator with protein kinase C in a manner sufficient to stimulate the synthesis of proteins sufficient to consolidate long term memory, wherein the PKC activator is provided in the form of a pharmaceutical composition comprising the PKC activator and a pharmaceutically acceptable carrier.

In one embodiment, the pharmaceutical composition further comprises a PKC inhibitor. In another embodiment, the PKC inhibitor is a compound that inhibits PKC in peripheral tissues. As used herein, "peripheral tissues" means tissues other than brain. In another embodiment, the PKC inhibitor is a compound that preferentially inhibits PKC in peripheral tissues. In another embodiment, the PKC inhibit is a compound that reduces myalgia associated with the administration of a PKC activator to subjects in need thereof. In another embodiment, the PKC inhibitor is a compound that reduces myalgia produced in a subject treated with a PKC activator. In another embodiment, the PKC inhibitor is a compound that increases the tolerable dose of a PKC activator. Specifically, PKC inhibitors include, for example, but are not limited to vitamin E, vitamin E analogs, and salts thereof; calphostin C; thiazolidinediones; ruboxistaurin, and combinations thereof. As used herein, "vitamin E" means α-tocopherol (5, 7, 8-trimethyltocol); β-tocopherol (5, 8-dimethyltocol; δ-tocopherol (8-methyltocal); and γ-tocopherol (7,8-dimethyltocol), salts and analogs thereof.

### BRIEF DESCRIPTION OF THE DRAWINGS

**Figure 1** depicts the effects of bryostatin on long term memory acquisition, and shows that animals trained sub-optimally, but treated with bryostatin, all demonstrate acquisitioned long-term memory.
**Figure 2** depicts the effects of bryostatin on long-term memory acquisition, and shows that randomized presentations of light and rotation, either with or without bryostatin, produced no conditioned response.
**Figure 3** depicts the effects of bryostatin on long-term memory acquisition, and shows that animals exposed to bryostatin for four hours on two successive days, followed by two training events (TE) on a third subsequent day, demonstrated acquisition of at least six days of long-term memory.
**Figure 4** depicts the effects of bryostatin on long term memory acquisition, and shows that animals exposed to bryostatin for four hours on three successive days, followed by two TE on a fourth subsequent day, demonstrated acquisition of at least ninety-six hours of long-term memory.
**Figure 5** depicts the effects of bryostatin on long term memory acquisition, and shows that exposure to bryostatin for 8 to 20 hours followed by two TE was not sufficient to acquire memory equivalent to that achieved after a 4-hour exposure to bryostatin.
**Figure 6** depicts the effects of bryostatin on long term memory acquisition, and shows that exposure to more than 1.0 ng/ml of bryostatin inhibits acquisition of long-term memory.
**Figure 7** depicts the effects of bryostatin and anisomycin on long-term memory acquisition, and shows that a single 4-hour exposure to bryostatin together with 2 TE produced long-term memory lasting hours that was entirely eliminated when anisomycin was present during bryostatin exposure.
**Figure 8** depicts the effects of bryostatin and lactacysteine, and shows that lactacysteine transformed the short-term memory produced by the single bryostatin exposure (followed by 2 TE) to long-term memory lasting many days.
**Figure 9** depicts the effects of PKC activation on calexcitin.
**Figure 10a** depicts the effect of bryostatin and training events on calexcitin immunostaining. The figure shows calexcitin increased within Type B cells with the number of training events.
**Figure 10b** depicts the effect of bryostatin alone calexcitin, as shown by immunostaining.
**Figure 11a** depicts the effect of 4-hour bryostatin exposure, on two consecutive days, followed 24 hours later by two training events, on the intensity of calexcitin. The figure shows that exposure to 4 hours of bryostatin on two consecutive days followed 24 hours later by 2 TEs are required to raise calexcitin levels to the amount associated with consolidated long-term memory.
**Figure 11b** depicts the effect of adding anisomycin after bryostatin exposure on calexcitin. The figure shows that anisomycin following 2 TE plus 3 days of 4 hour bryostatin exposures did not reduce the calexcitin immunostaining.
**Figure 12** depicts the effects of repeated 4-hour bryostatin exposure on PKC activity, as measured by histone phosphorylation in the cytosolic fraction. The figure shows bryostatin exposure on two successive days produces PKC activity significantly above control or baseline levels.
**Figure 13** depicts the effects of repeated 4-hour bryostatin exposure on PKC activity, as measured by histone phosphorylation in the membrane fraction. The figure shows bryostatin exposure on two successive days produces PKC activity significantly above control or baseline levels.
**Figure 14** depicts the effects of anisomycin on PKC activity. The figure shows that the presence of anisomycin during each of three successive days of bryostatin exposure reduced PKC activity in both cytosolic and membrane fractions.
**Figure 15** depicts the effects of bryostatin on membrane-bound PKC in hippocampal neurons. The figure shows that exposure of cultured hippocampal neurons to a single activating dose of bryostatin (0.28 nM) for 30 minutes produced a brief translocation of PKC from the cytosol to the particulate fraction (approx 60%) followed by a prolonged downregulation. A second exposure of up to four hours after the first exposure significantly attenuates the down regulation found four hours after a single bryostatin exposure.
**Figure 16** depicts the effects of repeated bryostatin exposure on PKC activity. The figure shows that a second exposure after a 2- to 4-hour delay eliminated the significant downregulation that a single 30-minute bryostatin exposure produced, and that if the second exposure was delayed until 4 hours after the first, activity was increased above baseline, to a degree that was significantly greater compared with a second exposure delivered after 2 hours or less.

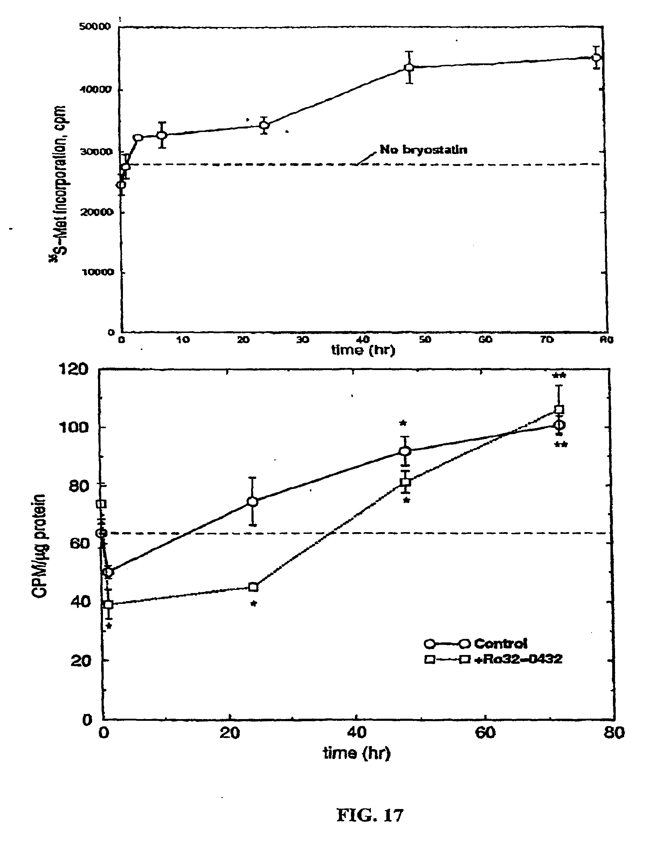

**Figure 17** depicts the effects of bryostatin on protein synthesis. Rat IGF-IR cells were incubated for 30 minutes with 0.28 nM bryostatin for incubation times ranging from 1 to 79 hours. [³⁵S]Methionine (9.1 µCi) was then added to the medium followed by analysis of radiolabel. A single 30-minute exposure to 0.28 nM bryostatin increased overall protein synthesis, as measured by the incorporation of [³⁵S]Methionine in the last half hour before collecting the neurons, by 20% within 24 hours, increasing to 60% by 79 hours after bryostatin exposure, but increasing significantly less in the presence of the PKC inhibitor Ro-32-0432.

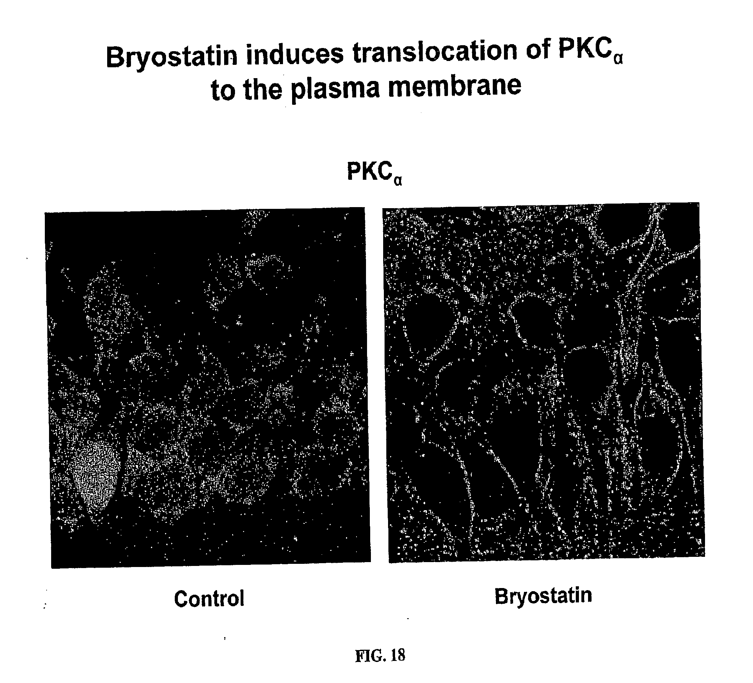

**Figure 18** depicts the induction of PKCα translocation to the plasma membrane of neuronal cells.

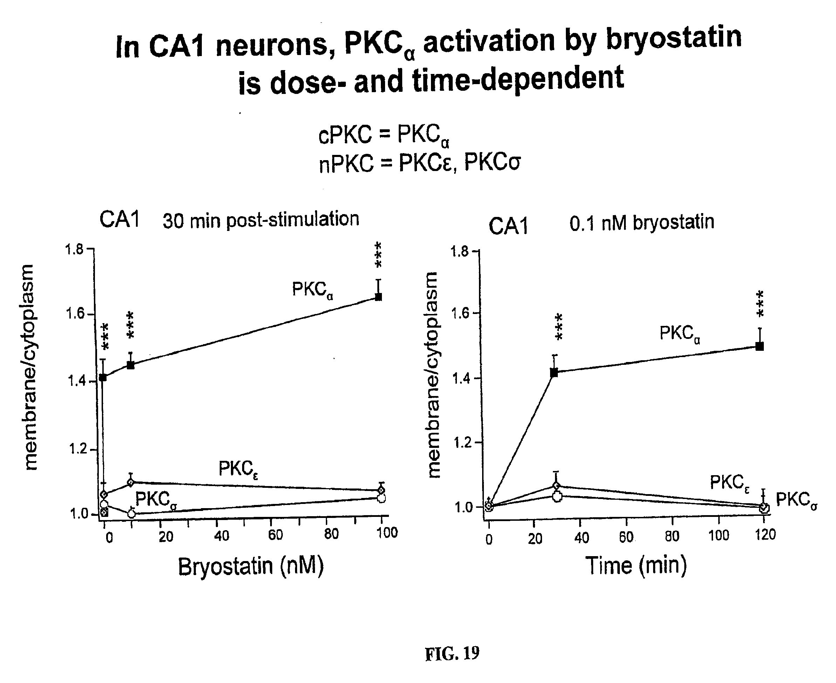

**Figure 19** depicts the dose and time dependence of PKCα in CA1 neurons.

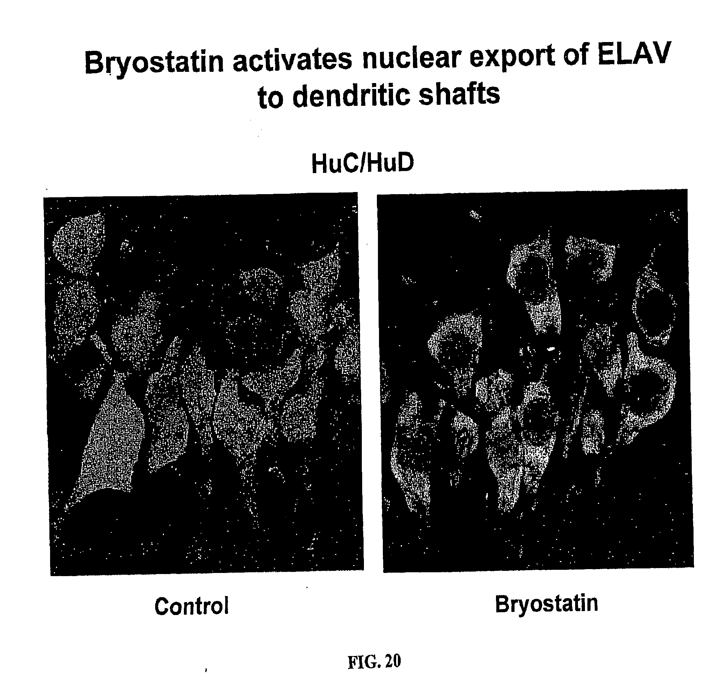

**Figure 20** depicts the bryostatin-mediated nuclear export of ELAV to dendritic shafts.

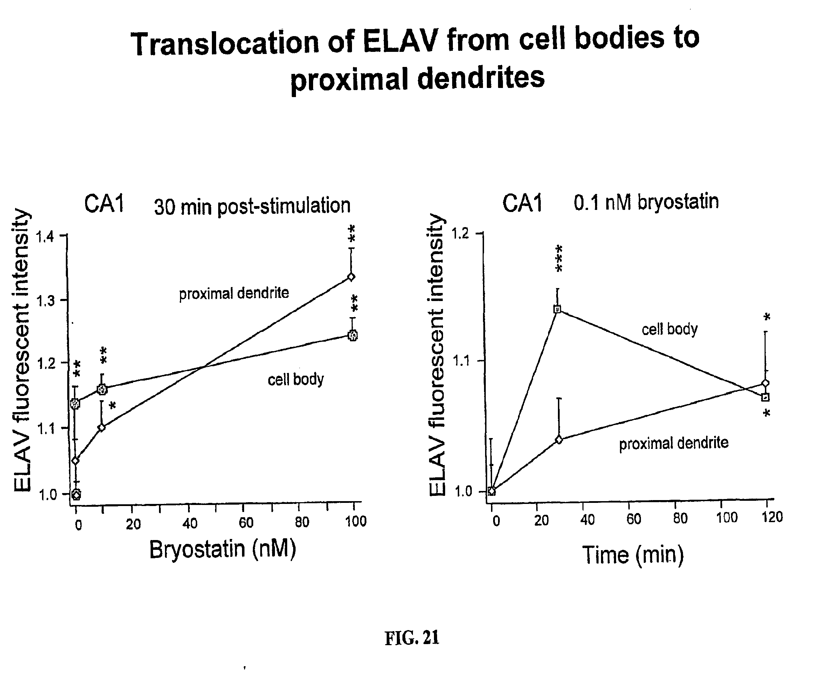

**Figure 21** depicts the dose and time dependence of the bryostatin-mediated nuclear export of ELAV to dendritic shafts.

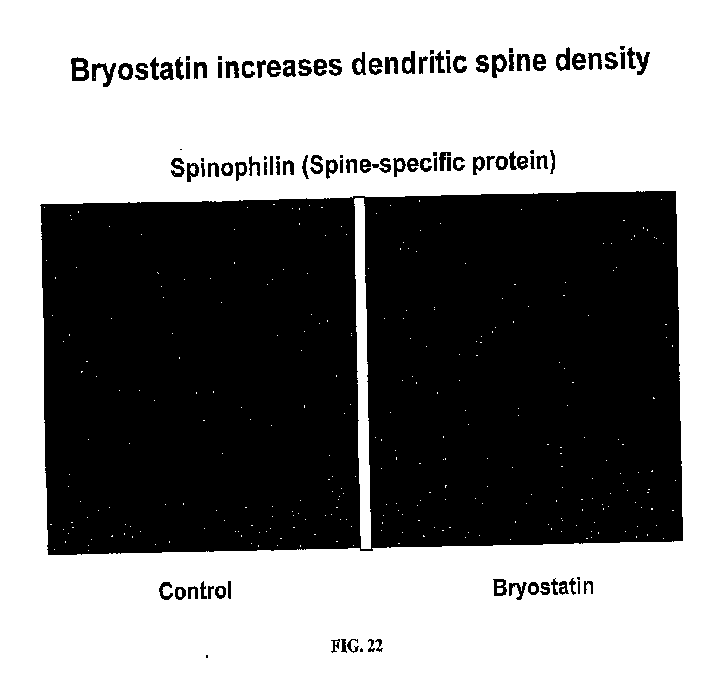

**Figure 22** depicts the bryostatin-mediated increase in dendritic spine density, as measured by labeling of the spine-specific protein, spinophilin.

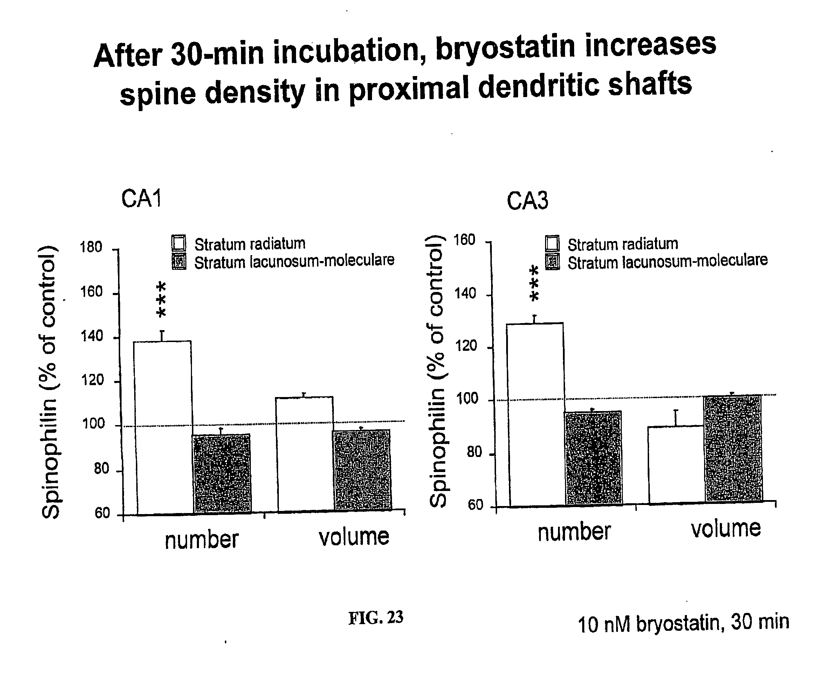

**Figure 23** depicts the bryostatin-mediated increase in dendritic spine density in proximal dendritic shafts, after a 30-minute exposure of CA1 and CA3 neurons to bryostatin.

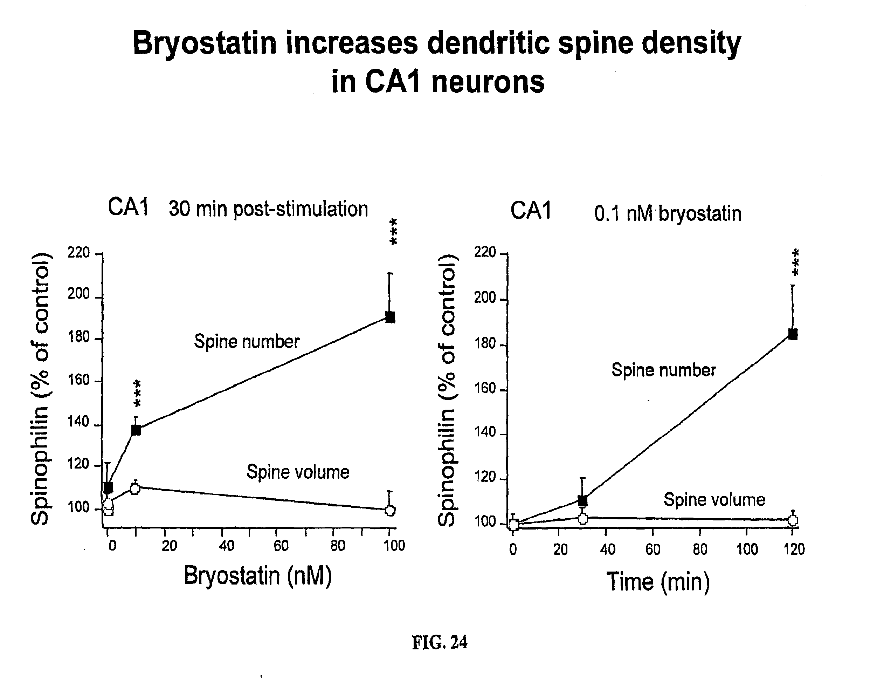

**Figure 24** depicts the dose and time dependence of the bryostatin-mediated increase in spine number in CA1 neurons.

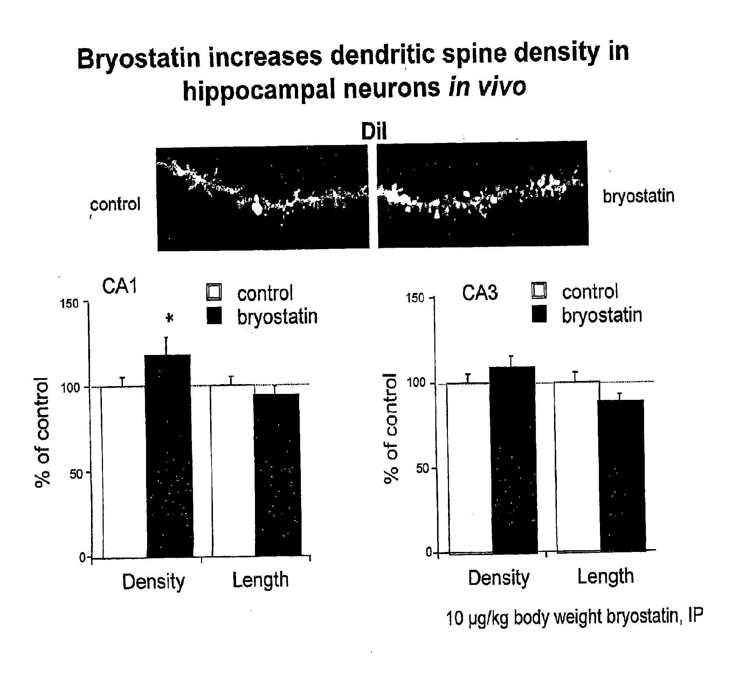

**Figure 25** depicts the bryostatin-mediated increase in spine number in CA1 and CA3 hippocampal neurons, *in vivo.*
**Figure 26** depicts the effects of bryostatin on learning and the retention of memory. Asterisks are significantly different from swim controls (**, p<.01; **, p<.001). In probe tests, Maze+Bryo is significantly different from Maze and from Maze+Bryo+RO (p<.05).
**Figure 27** depicts the effects of bryostatin on dendritic spines of rats trained in a spatial maze task. Asterisks indicate significantly differences from naive controls (*, p<.05; **, p<.001).
**Figure 28** depicts the effects of bryostatin on mushroom spines. Asterisks are significantly different from naive controls (*, p<.05; **, p<.001).
**Figure 29** depicts different effects of bryostatin on pre- and postsynaptic structures. Asterisks indicate significantly differences from naive controls (*, p<.05; **, p<.01; ***, p<.001).
**Figure 30** depicts the mechanism of increased spine density by PKC activation. Yellow arrows show the plasma membrane. White arrows depict the CA1 neurons.
**Figure 31** depicts the mechanism of increased spine density by PKC activation. Asterisks indicate significantly differences from naive controls (*, p<.05; **, p<.01; ***, p<.001).

### DETAILED DESCRIPTION OF THE INVENTION

### 1. Definitions

As used herein, "upregulating" or "upregulation" means increasing the amount or activity of an agent, such as PKC protein or transcript, relative to a baseline state, through any mechanism including, but not limited to increased transcription, translation and/or increased stability of the transcript or protein product.

As used herein, "down regulating" or " down regulation" means decreasing the amount or activity of an agent, such as PKC protein or transcript, relative to a baseline state, through any mechanism including, but not limited to decreased transcription, translation and/or decreased stability of the transcript or protein product.

As used herein, the term "pharmaceutically acceptable carrier" means a chemical composition, compound, or solvent with which an active ingredient may be combined and which, following the combination, can be used to administer the active ingredient to a subject. As used herein, "pharmaceutically acceptable carrier" includes, but is not limited to, one or more of the following: excipients; surface active agents; dispersing agents; inert diluents; granulating and disintegrating agents; binding agents; lubricating agents; preservatives; physiologically degradable compositions such as gelatin; aqueous vehicles and solvents; oily vehicles and solvents; suspending agents; dispersing or wetting agents; emulsifying agents, demulcents; buffers; salts; thickening agents; fillers; antioxidants; stabilizing agents; and pharmaceutically acceptable polymeric or hydrophobic materials and other ingredients known in the art and described, for example in Genaro, ed. (1985) Remington's Pharmaceutical Sciences Mack Publishing Co., Easton, Pa., which is incorporated herein by reference.

The formulations of the pharmaceutical compositions described herein may be prepared by any method known or hereafter developed in the art of pharmacology. In general, such preparatory methods include the step of bringing the active ingredient into association with a carrier or one or more other accessory ingredients, and then, if necessary or desirable, shaping or packaging the product into a desired single- or multi-dose unit.

Although the descriptions of pharmaceutical compositions provided herein are principally directed to pharmaceutical compositions which are suitable for ethical administration to humans, it will be understood by the skilled artisan that such compositions are generally suitable for administration to animals of all sorts. Modification of pharmaceutical compositions suitable for administration to humans in order to render the compositions suitable for administration to various animals is well understood, and the ordinarily skilled veterinary pharmacologist can design and perform such modification with merely ordinary, if any, experimentation. Subjects to which administration of the pharmaceutical compositions of the invention is contemplated include, but are not limited to, humans and other primates, and other mammals.

### 2. Alzheimer's Disease

Alzheimer's disease is associated with extensive loss of specific neuronal subpopulations in the brain with memory loss being the most universal symptom. (Katzman (1986) New England Journal of Medicine 314: 964). Alzheimer's disease is well characterized with regard to neuropathological changes. However, abnormalities have been reported in peripheral tissue supporting the possibility that Alzheimer's disease is a systematic disorder with pathology of the central nervous system being the most prominent. (Connolly (1998) Review, TiPS Col. 19: 171-77). For a discussion of Alzheimer's disease links to a genetic origin and chromosomes 1, 14, and 21 see St. George-Hyslop et al. (1987) Science 235: 885; Tanzi et al. Review, Neurobiology of Disease 3:159-168; Hardy (1996) Acta Neurol Scand: Supplement 165: 13-17.

Individuals with Alzheimer's disease are characterized by progressive memory impairments, loss of language and visuospatial skills and behavior deficits (McKhann et al. (1986) Neurology 34: 939-944). The cognitive impairment of individuals with Alzheimer's disease is the result of degeneration of neuronal cells located in the cerebral cortex, hippocampus, basal forebrain and other brain regions. Histologic analyzes of Alzheimer's disease brains obtained at autopsy demonstrated the presence of neurofibrillary tangles (NFT) in perikarya and axons of degenerating neurons, extracellular neuritic (senile) plaques, and amyloid plaques inside and around some blood vessels of affected brain regions. Neurofibrillary tangles are abnormal filamentous structures containing fibers (about 10 nm in diameter) that are paired in a helical fashion, therefore also called paired helical filaments. Neuritic plaques are located at degenerating nerve terminals (both axonal and dendritic), and contain a core compound of amyloid protein fibers. In summary, Alzheimer's disease is characterized by certain neuropathological features including intracellular neurofibrillary tangles, primarily composed of cytoskeletal proteins, and extracellular parenchymal and cerebrosvascular amyloid. Further, there are now methods in the art of distinguishing between Alzheimer's patents, normal aged people, and people suffering from other neurodegenerative diseases, such as Parkinson's, Huntington's chorea, Wernicke-Korsakoff or schizophrenia further described for instance in U.S. Patent 5,580,748 and U.S. Patent 6,080,582.

While cellular changes leading to neuronal loss and the underlying etiology of the disease remain under investigation the importance of APP metabolism is well established. The two proteins most consistently identified in the brains of patients with Alzheimer's disease to play a role in the physiology or pathophysiology of brain are β-amyloid and tau. *(See* Selkoe (2001) Physiological Reviews. 81:2). A discussion of the defects in β-amyloid protein metabolism and abnormal calcium homeostasis and/or calcium activated kinases. (Etcheberrigaray et al. Alzheimer's Reports Vol. Nos. 3, 5 & 6 pp 305-312; Webb et al. (2000) British Journal of Pharmacology 130: 1433-52).

Alzheimer's disease (AD) is a brain disorder characterized by altered protein catabolism. Altered protein phosphorylation has been implicated in the formation of the intracellular neurofibrillary tangles found in Alzheimer's disease. The processing of the amyloid precursor protein (APP) determines the production of fragments that later aggregate forming the amyloid deposits characteristic of Alzheimer's disease (AD), known as senile or AD plaques. A central feature of the pathology of Alzheimer's disease is the deposition of amyloid protein within plaques. Thus, APP processing is an early and key pathophysiological event in AD.

Three alternative APP processing pathways have been identified. The previously termed "normal" processing involves the participation of an enzyme that cleaves APP within the Aβ sequence at residue Lys16 (or between Lys16 and Leu17; APP770 nomenclature), resulting in non-amyloidogenic fragments: a large N-terminus ectodomain and a small 9 kDa membrane bound fragment. This enzyme, yet to be fully identified, is known as α-secretase. Two additional secretases participate in APP processing. One alternative pathway involves the cleavage of APP outside the Aβ domain, between Met671 and Asp672 (by β-secretase) and the participation of the endosomal-lysomal system. An additional cleavage site occurs at the carboxyl-terminal end of the Aβ portion, within the plasma membrane after amino acid 39 of the Aβ peptide. The secretase (γ) action produces an extracellular amino acid terminal that contains the entire Aβ sequence and a cell-associated fragment of ∼6kDa. Thus, processing by β and γ secretases generate potential amyloidogenic fragments since they contain the complete Aβ sequence. Several lines of evidence have shown that all alternative pathways occur in a given system and that soluble Aβ may be a "normal product." However, there is also evidence that the amount of circulating Aβ in CSF and plasma is elevated in patients carrying the "Swedish" mutation. Moreover, cultured cells transfected with this mutation or the APP₇₁₇ mutation, secrete larger amounts of Aβ. More recently, carriers of other APP mutations and PS1 and PS2 mutations have been shown to secrete elevated amounts of a particular form, long (42-43 amino acids) Aβ.

Therefore, although all alternative pathways may occur normally, an imbalance favoring amyloidogenic processing occurs in familial and perhaps sporadic AD. These enhanced amyloidogenic pathways ultimately lead to fibril and plaque formation in the brains of AD patients. Thus, intervention to favor the non-amyloidogenic, α-secretase pathway effectively shifts the balance of APP processing towards a presumably non-pathogenic process that increases the relative amount of sAPP compared with the potentially toxic Aβ peptides.

The PKC isoenzymes provides a critical, specific and rate limiting molecular target through which a unique correlation of biochemical, biophysical, and behavioral efficacy can be demonstrated and applied to subjects to improve cognitive ability.

Further with regard to normal and abnormal memory both K⁺ and Ca²⁺ channels have been demonstrated to play key roles in memory storage and recall. For instance, potassium channels have been found to change during memory storage. (Etcheberrigaray et al. (1992) Proc. Natl. Acad. Sci. 89: 7184; Sanchez-Andres et al. (1991) Journal of Neurobiology 65: 796; Collin et al. (1988) Biophysics Journal 55: 955; Alkon et al. (1985) Behavioral and Neural Biology 44: 278; Alkon (1984) Science 226: 1037). This observation, coupled with the almost universal symptom of memory loss in Alzheimer's patents, led to the investigation of potassium channel function as a possible site of Alzheimer's disease pathology and the effect of PKC modulation on cognition.

### 3. Protein Kinase C and Alzheimer's Disease

PKC was identified as one of the largest gene families of non-receptor serine-threonine protein kinases. Since the discovery of PKC in the early eighties by Nishizuka and coworkers (Kikkawa et al. (1982) J. Biol. Chem. 257: 13341), and its identification as a major receptor of phorbol esters (Ashendel et al. (1983) Cancer Res., 43: 4333), a multitude of physiological signaling mechanisms have been ascribed to this enzyme. The intense interest in PKC stems from its unique ability to be activated *in vitro* by calcium and diacylglycerol (and its phorbol ester mimetics), an effector whose formation is coupled to phospholipid turnover by the action of growth and differentiation factors.

The PKC gene family consists presently of 11 genes which are divided into four subgrounds: 1) classical PKCα, β₁, β₂ (β₁ and β₂ are alternatively spliced forms of the same gene) and γ, 2) novel PKCδ, ε, η and θ, 3) atypical PKCζ, λ, η and τ and 4) PKCµ. PKCµ resembles the novel PKC isoforms but differs by having a putative transmembrane domain (reviewed by Blohe et al. (1994) Cancer Metast. Rev. 13: 411; Ilug et al. (1993) Biochem J. 291: 329; Kikkawa et al. (1989) Ann. Rev. Biochem. 58: 31). The α, β₁, β₂, and y isoforms are Ca², phospholipid and diacylglycerol-dependent and represent the classical isoforms of PKC, whereas the other isoforms are activated by phospholipid and diacylglycerol but are not dependent on CA²⁺. All isoforms encompass 5 variable (V1-V5) regions, and the α, β, γ isoforms contain four (C1-C4) structural domains which are highly conserved. All isoforms except PKCα, β and γ lack the C2 domain, and the λ, η and isoforms also lack nine of two cysteine-rich zinc finger domains in C1 to which diacylglycerol binds. The C1 domain also contains the pseudo-substrate sequence which is highly conserved among all isoforms, and which serves an auto-regulatory function by blocking the substrate-binding site to produce an inactive conformation of the enzyme (House et al., (1987) Science 238: 1726).

Because of these structural features, diverse PKC isoforms are thought to have highly specialized roles in signal transduction in response to physiological stimuli (Nishizuka (1989) Cancer 10: 1892), as well as in neoplastic transformation and differentiation (Glazer (1994) Protein Kinase C. J.F. Kuo, ed., Oxford U. Press (1994) at pages 171-198). For a discussion of known PKC modulators see PCT/US97/08141, U.S. Patent Nos. 5,652,232; 6,043,270; 6,080,784; 5,891,906; 5,962,498; 5,955,501; 5,891,870 and 5,962,504.

In view of the central role that PKC plays in signal transduction, PKC has proven to be an exciting target for the modulation of APP processing. It is well established that PKC plays a role in APP processing. Phorbol esters for instance have been shown to significantly increase the relative amount of non-amyloidogenic soluble APP (sAPP) secreted through PKC activation. Activation of PKC by phorbol ester does not appear to result in a direct phosphorylation of the APP molecule, however. Irrespective of the precise site of action, phorbol-induced PKC activation results in an enhanced or favored α-secretase, non-amyloidogenic pathway. Therefore PKC activation is an attractive approach for influencing the production of non-deleterious sAPP and even producing beneficial sAPP and at the same time reduce the relative amount of Aβ peptides. Phorbol esters, however, are not suitable compounds for eventual drug development because of their tumor promotion activity. (Ibarreta et al. (1999) NeuroReport Vol. 10, No. 5&6, pp 1034-40).

The present inventors have also observed that activation of protein kinase C favors the α-secretase processing of the Alzheimer's disease (AD) amyloid precursor protein (APP), resulting in the generation of non-amyloidogenic soluble APP (sAPP). Consequently, the relative secretion of amyloidogenic A₁₋₄₀ and A₁₋₄₂₍₃₎ is reduced. This is particularly relevant since fibroblasts and other cells expressing APP and presenilin AD mutations secrete increased amounts of total Aβ and/or increased ratios of A₁₋₄₂₍₃₎/₁₋₄₀. Interesting, PKC defects have been found in AD brain (α and β isoforms) and in fibroblasts (α-isoform) from AD patients.

Studies have shown that other PKC activators (i.e. benzolactam) with improved selectivity for the α, β and γ isoforms enhance sAPP secretion over basal levels. The sAPP secretion in benzolactam-treated AD cells was also slightly higher compared to control benzolactam-treated fibroblasts, which only showed significant increases of sAPP secretion after treatment with 10 µM BL. It was further reported that staurosporine (a PKC inhibitor) eliminated the effects of benzolactam in both control and AD fibroblasts while related compounds also cause a ∼3-fold sAPP secretion in PC12 cells. The present inventors have found that the use of bryostatin as a PKC activators to favor non-amyloidogenic APP processing is of particular therapeutic value since it is non-tumor promoting and already in stage II clinical trials.

Alterations in PKC, as well alterations in calcium regulation and potassium (K⁺) channels are included among alterations in fibroblasts in Alzheimer's disease (AD) patients. PKC activation has been shown to restore normal K⁺ channel function, as measured by TEA-induced [Ca²⁺] elevations. Further patch-clamp data substantiates the effect of PKC activators on restoration of 113psK⁺ channel activity. Thus PKC activator-based restoration of K⁺ channels has been established as an approach to the investigation of AD pathophysiology, and provides a useful model for AD therapeutics. (See, pending U.S. Application Serial No. 09/652,656, which is incorporated herein by reference in its entirety.)

Of particular interest are macrocyclic lactones (i.e. bryostatin class and neristatin class) that act to stimulate PKC. Of the bryostatin class compounds, bryostatin-1 has been shown to activate PKC and proven to be devoid of tumor promotion activity. Bryostatin-1, as a PKC activator, is also particularly useful since the dose response curve of bryostatin-1 is biphasic. Additionally, bryostatin-1 demonstrates differential regulation of PKC isozymes, including PKCα, PKCδ, and PKCε. Bryostatin-1 has undergone toxicity and safety studies in animals and humans and is actively being investigated as an anti-cancer agent. Bryostatin-1's use in the studies has determined that the main adverse reaction in humans is myalgia, limiting the maximum dose to 40 mg/m². The present invention has utilized concentrations of 0.1 nM of bryostatin-1 to cause a dramatic increase of sAPP secretion. Bryostatin-1 has been compared to a vehicle alone and to another PKC activator, benzolactam (BL), used at a concentration 10,000 times higher. Bryostatin is currently in clinical trials as an anti-cancer agent. The bryostatins are known to bind to the regulatory domain of PKC and to activate the enzyme. Bryostatin is an example of isozyme-selective activators of PKC. Compounds in addition to bryostatins have been found to modulate PKC. (*See, for example,* WO 97/43268; incorporated herein by reference in its entirety).

Macrocyclic lactones, and particularly bryostatin-1 is described in U.S. Patent 4,560,774 (incorporated herein by reference in its entirety). Macrocyclic lactones and their derivatives are described elsewhere in the art for instance in U.S. Patent 6,187,568, U.S. Patent 6,043,270, U.S. Patent 5,393,897, U.S. Patent 5,072,004, U.S. Patent 5,196,447, U.S. Patent 4,833,257, and U.S. Patent 4,611,066 (each of which are incorporated herein by reference in their entireties). The above patents describe various compounds and various uses for macrocyclic lactones including their use as an anti-inflammatory or anti-tumor agent. Other discussions regarding bryostatin class compounds can be found in: Szallasi et al. (1994) Differential Regulation of Protein Kinase C Isozymes by Bryostatin 1 and Phorbol 12-Myristate 13-Acetate in NIH 3T3 Fibroblasts, Journal of Biological Chemistry 269(3): 2118-24; Zhang et al. (1996) Preclinical Pharmacology of the Natural Product Anticancer Agent Bryostatin 1, an Activator of Protein Kinase C, Cancer Research 56: 802-808; Hennings et al. (1987) Bryostatin 1, an activator of protein kinase C, inhibits tumor promotion by phorbol esters in SENCAR mouse skin, Carcinogenesis 8(9): 1343-46; Varterasian et al. (2000) Phase II Trial of Bryostatin 1 in Patients with Relapse Low-Grade Non-Hodgkin's Lymphoma and Chronic Lymphocytic Leukemia, Clinical Cancer Research 6: 825-28; and Mutter et al. (2000) Review Article: Chemistry and Clinical Biology of the Bryostatins, Bioorganic & Medicinal Chemistry 8: 1841-1860 (each of which is incorporated herein by reference in its entirety).

Myalgia is the primary side effect that limits the tolerable dose of a PKC activator. For example, in phase II clinical trials using bryostatin-1, myalgia was reported in 10 to 87% of all treated patients. (Clamp et al. (2002) Anti-Cancer Drugs 13: 673-683). Doses of 20 µg/m² once per week for 3 weeks were well tolerated and were not associated with myalgia or other side effects. (Weitman et al. (1999) Clinical Cancer Research 5: 2344-2348). In another clinical study, 25 µg/m² of bryostatin-1 administered once per week for 8 weeks was the maximum tolerated dose. (Jayson et al. (1995) British J. of Cancer 72(2): 461-468). Another study reported that 50 µg/m² (a 1 hour i.v. infusion administered once every 2 weeks for a period of 6 weeks) was the maximum-tolerated dose. (Prendville et al. (1993) British J. of Cancer 68(2): 418-424). The reported myalgia was cumulative with repeated treatments of bryostatin-1 and developed several days after initial infusion. *Id*. The deleterious effect of myalgia on a patient's quality of life was a contributory reason for the discontinuation of bryostatin-1 treatment. *Id*. The etiology of bryostatin-induced myalgia is uncertain. *Id*.

The National Cancer Institute has established common toxicity criteria for grading myalgia. Specifically, the criteria are divided into five categories or grades. Grade 0 is no myalgia. Grade 1 myalgia is characterized by mild, brief pain that does not require analgesic drugs. In Grade 1 myalgia, the patient is fully ambulatory. Grade 2 myalgia is characterized by moderate pain, wherein the pain or required analgesics interfere with some functions, but do not interfere with the activities of daily living. Grade 3 myalgia is associated with severe pain, wherein the pain or necessary analgesics severely interfere with the activities of daily living. Grade 4 myalgia is disabling.

The compositions of the present invention increase the tolerable dose of the PKC activator administered to a patient and/or ameliorate the side effects associated with PKC activation by attenuating the activation of PKC in peripheral tissues. Specifically, PKC inhibitors inhibit PKC in peripheral tissues or preferentially inhibit PKC in peripheral tissues. Vitamin E, for example, has been shown to normalize diacylglycerol-protein kinase C activation in the aorta of diabetic rats and cultured rat smooth muscle cells exposed to elevated glucose levels. (Kunisaki et al. (1994) Diabetes 43(11): 1372-1377). In a double-blind trial of vitamin E (2000 IU/day) treatment in patients suffering from moderately advanced Alzheimer's Disease, it was found that vitamin E treatment reduced mortality and morbidity, but did not enhance cognitive abilities. (Burke et al, (1999) Post Graduate Medicine 106(5): 85-96).

Macrocyclic lactones, including the bryostatin class were originally derived from *Bigula neritina L.* While multiple uses for macrocyclic lactones, particularly the bryostatin class are known, the relationship between macrocyclic lactones and cognition enhancement was previously unknown.

The examples of the compounds that may be used in the present invention include macrocyclic lactones (i.e. bryostatin class and neristatin class compounds). While specific embodiments of these compounds are described in the examples and detailed description, it should be understood that the compounds disclosed in the references and derivatives thereof could also be used for the present compositions and methods.

As will also be appreciated by one of ordinary skill in the art, macrocyclic lactone compounds and their derivatives, particularly the bryostatin class, are amenable to combinatorial synthetic techniques and thus libraries of the compounds can be generated to optimize pharmacological parameters, including, but not limited to efficacy and safety of the compositions. Additionally, these libraries can be assayed to determine those members that preferably modulate α-secretase and/or PKC.

Synthetic analogs of bryostatin are also contemplated by the present invention. Specifically, these analogues retain the orientation of the C1-, C19-, C26-oxygen recognition domain as determined by NMR spectroscopic comparison with bryostatin and various degrees of PKC-binding affinity. The bryostatin analogues disclosed and described in U.S. Patent No. 6,624,189 (incorporated herein by reference in its entirety) may also be used in the methods of the present invention. Specifically, the bryostatin analogues described by the genus of Formula I of U.S. Patent No. 6,624,189 (column 3, lines 35-66) and the species of formulas II-VII and 1998a and 1998b (column 8, lines 28-60) of U.S. Patent No. 6,624,189 are PKC activators suitable for use in the methods of the present invention.

There still exists a need for the development of methods for the treatment for improved overall cognition, either through a specific characteristic of cognitive ability or general cognition. There also still exists a need for the development of methods for the improvement of cognitive enhancement whether or not it is related to specific disease state or cognitive disorder. The methods and compositions of the present invention fulfill these needs and will greatly improve the clinical treatment for Alzheimer's disease and other neurodegenerative diseases, as well as, provide for improved cognitive enhancement. The methods and compositions also provide treatment and/or enhancement of the cognitive state through the modulation of α-secretase.

### 4. Protein Kinase C Activation and Synaptic Plasticity

Activiation of protein kinase C stimulates synaptic plasticity. Synaptic plasticity is a term used to describe the ability of the connection, or synapse, between two neurons to change in strength. Several mechanisms cooperate to achieve synaptic plasticity, including changes in the amount of neurotransmitter released into a synapse and changes in how effectively cells respond to those neurotransmitters (Gaiarsa et al., 2002). Because memory is produced by interconnected networks of synapses in the brain, synaptic plasticity is one of the important neurochemical foundations of learning and memory.

Changes in dendritic spine density forms the basis of synaptic plasticity. Changes in dendritic spine density play a role in many brain functions, including learning and memory. Long-term memory, for example, is mediated, in part, by the growth of new dendritic spines to reinforce a particular neural pathway. By strengthening the connection between two neurons, the ability of the presynaptic cell to activate the postsynaptic cell is enhanced.

A dendritic spine is a small (sub-micrometre) membranous extrusion that protrudes from a dendrite and forms one half of a synapse. Typically spines have a bulbous head (the spine head) which is connected to the parent dendrite through a thin spine neck. Dendritic spines are found on the dendrites of most principal neurons in the brain. Spines are categorized according to shape as, for example, mushroom spines, thin spines and stubby spines. Electron microscopy shows a continuum of shapes between these categories. There is some evidence that differently shaped spines reflect different developmental stages and strengths of a synapse. Laser scanning and confocal microscopy have been used to show changes in dendritic spine properties, including spine size and density. Using the same techniques, time-lapse studies in the brains of living animals have shown that spines come and go, with the larger mushroom spines being the most stable over time.

Several proteins are markers for dendritic spine formation. Spinophilin, for example, is highly enriched in dendritic spines and has been shown to regulate the formation and function of dendritic spines. ELAV proteins are one of the earliest markers of neuronal differentiation. ELAV proteins are generally involved in the post-transcriptional regulation of gene expression.

### EXAMPLES

### Example 1: Behavioral Pharmacology

Specimens of *Hermissenda Crassicornis* were maintained in artificial sea water (ASW) at 15° for three days in perforated 50-ml conical centrifuge tubes before starting experiments. Bryostatin, purified from the marine bryozoan *Bugula neritinα,* was dissolved in EtOH and diluted to its final concentration in ASW. Animals were incubated with bryostatin in ASW for 4 hr, then rinsed with normal ASW. For selected experiments lactacysteine (10µM) or anisomycin was added to the ASW.

Bryostatin effects on *Hermissenda* behavior and biochemistry were produced by adding the drug to the bathing medium within an 8 cm long, 1 cm diameter test tube housing each individual animal.

### Example 2: Immunostaining Methods

Following experimental treatments and testing, animals were rapidly decapitated, the central nervous systems (CNS) removed and then fixed in 4% para-formaldehyde in 20 mM Tris-buffered (pH 8) natural seawater (NSW; 0.2µm micropore-filtered). The CNSs were then embedded in polyester wax (20), sectioned (6 µm) and immunostained using a biotinylated secondary antibody coupled to avidin-bound microperoxidase (ABC method, Vector), Aminoethylcarbazole (AEC) was used as the chromogen. The primary polyclonal antibody (designated 25U2) was raised in rabbits from the full length calexcitin protein extracted from squid optic lobes. Gray-scale intensity measures were done from digital photomicrographs on circumscribed cytoplasmic areas of the B-photoreceptors minus the same background area (non-staining neuropile).

### Example 3: Protein Kinase C Assay

Cells were homogenized by sonication (5 sec, 25W) in 100µl of 10 mM Tris-HCL pH 7.4 buffer containing 1 mM EGTA, 1 mM PMSF, and 50 mM NaF. Homogenate was transferred to a polyallomer centrifuge tube and was centrifuged at 100,000xg for 10 min at 4°. The supernatant was removed and immediately frozen on dry ice. The particulate fraction was resuspended by sonication in 100µl of the same buffer and stored at -80°. To measure PKC, 10µl of cytosol or particulate fraction was incubated for 15 min at 37° in the presence of 10µM histones, 4.89 mM CaCl₂, 1.2µg/µl phosphatidyl-L-serine, 0.18µg/µl 1.2-dioctanoyl-sn-glycerol, 10 mM MgCl₂, 20 mM HEPES (pH 7.4), 0-8 mM EDTA, 4 mM EGTA, 4% glycerol, 8 µg/ml aprotinin, 8 µg/ml leupeptin, and 2 mM benzamidine. 0.5 µCi [γ³²-P]ATP was added and ³²P-phosphoprotein formation was measured by adsorption onto phosphocellulose as described previously (25). This assay was used with slight adjustments for either *Hermissenda* nervous system homogenates or cultured mammalian neuron homogenates

### Example 4: Cell Culture

Rat hippocampal H19-7/IGF-IR cells (ATCC) were plated onto poly-L-lysine coated plates and grown at 35° in DMEM/10% FCS for several days until approx. 50% coverage was obtained. The cells were then induced to differentiate into a neuronal phenotype by replacing the medium with 5 ml N2 medium containing 10 ng/ml basic fibroblast growth factor and grown in T-25 flasks at 39°C (26). Various concentrations of bryostatin (0.01-1.0 nM) were then added in 10µl aqueous solution. After a specified interval, the medium was removed and the cells were washed with PBS, removed by gentle scraping, and collected by centrifugation at 1000 rpm for 5 min.

### Example 5: Behavioral Conditioning

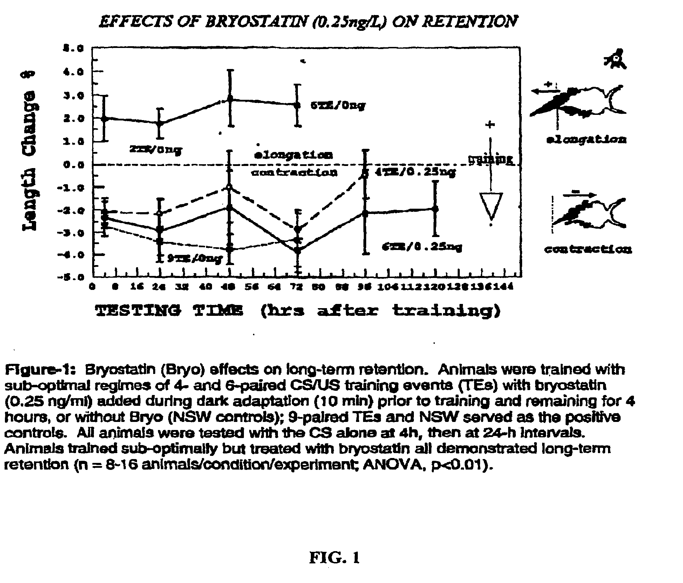

Pavlovian conditioning of *Hermissenda* involves repeated pairings of a neutral stimulus, light, with an unconditioned stimulus, orbital shaking. (*See*, Lederhendler *et al.* (24) and Epstein *et al.* (6)). A rotation/shaking stimulus excites the statocyst hair cells and thereby elicits an unconditioned response: a brisk contraction of the muscular undersurface called a foot, accompanied by adherence or "clinging" to the surface that supports the foot. Before conditioning, light elicits a weakly positive phototaxis accompanied by lengthening of the foot. After sufficient light-rotation pairings, light no longer elicits phototaxis, but instead elicits a new response (24): the "clinging" and foot shortening previously elicited only by the unconditional stimulus (Fig. 1). Thus, the meaning of the unconditioned stimulus, rotation or orbital shaking, has been transferred to the conditioned stimulus and is manifested by a light-elicited foot contraction-a negative change of foot length. This conditioned response to light can last for weeks, is not produced by randomized light and rotation, is stimulus-specific, and shares the other defining characteristics of mammalian Pavlovian Conditioning.

### Example 6: Bryostatin-induced prolongation of associative memory

Pavlovian conditioning of *Hermissenda* has well-defined training parameters that produce progressively longer-lasting retention of the learned conditioned response. Two training events (2 TE) of paired light and orbital shaking (see "Methods"), for example, induce a learned conditioned response (light-elicited foot contraction or shortening) that persists without drug treatment for approximately 7 minutes. Four to six training events (4-6 TE) induce a conditioned response that persists up to several hours, but disappears approximately by 1 day after training. Nine TE produces long-term associative memory lasting many days and often up to two weeks.

Animals were trained with sub-optimal regimes of 4- and 6-paired CS/US training events (TEs) with bryostatin (0.25 ng/ml) added during dark adaptation (10 min) prior to training and remaining for 4 hours, or without Bryo (NSW controls); 9-paired TEs and NSW served as the positive controls. All animals were tested with the CS alone at 4h, then at 24-h intervals. Animals trained sub-optimally but treated with bryostatin all demonstrated long-term retention (n = 8-16 animals/condition/experiment; ANOVA, p<0.01).

Two TE plus bryostatin produced memory retention lasting hours (vs. minutes without bryostatin), 4 TEs plus bryostatin extended retention beyond 24 hours (Fig. 1), and 6 TE plus bryostatin produced retention lasting 1 week or longer.

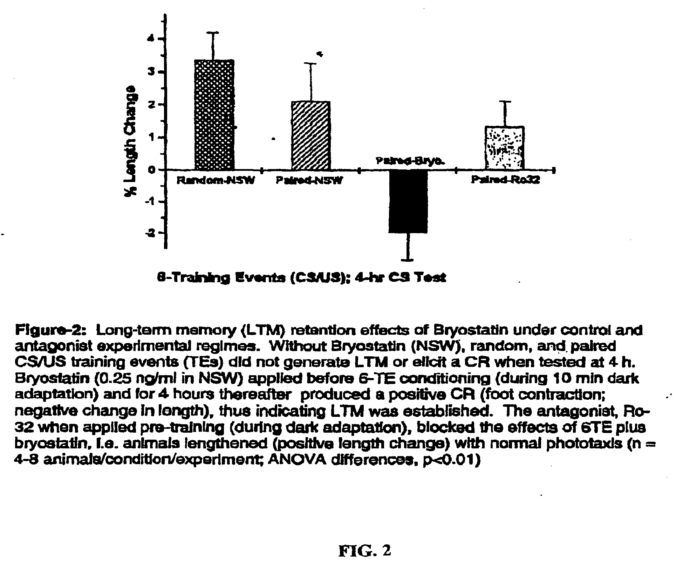

Without Bryostatin (NSW), random, and paired CS/US training events (TEs) did not generate LTM or elicit a CR when tested at 4 h. Bryostatin (0.25 ng/ml in NSW) applied before 6-TE conditioning (during 10 min dark adaptation) and for 4 hours thereafter produced a positive CR (foot contraction; negative change in length), thus indicating LTM was established. The antagonist, Ro-32 when applied pre-training (during dark adaptation), blocked the effects of 6 TE plus bryostatin, i.e. animals lengthened (positive length change) with normal phototaxis (n = 4-8 animals/condition/experiment; ANOVA differences, p<0.01). Randomized presentations of light and rotation, with or without bryostatin, produced no conditioned response (Fig. 2), i.e., light-elicited foot-contraction. Thus, bryostatin during and immediately following training prolonged memory retention with sub-optimal training trials.

### Example 7: Pre-exposure to bryostatin on days before trajnine enhances memory acquisition

Previous measurements (15, 17) have indicated that learning-induced PKC association with neuronal membranes (*i.e*., translocation) can be sustained. Rabbit nictitating membrane conditioning, rat spatial maze learning, maze learning, and rat olfactory discrimination learning have all been found to be accompanied by PKC translocation that lasts for days following training. *Hermissenda* conditioning was followed for at least one day after training by PKC translocation that could be localized in single, identifiable Type B cells (15).

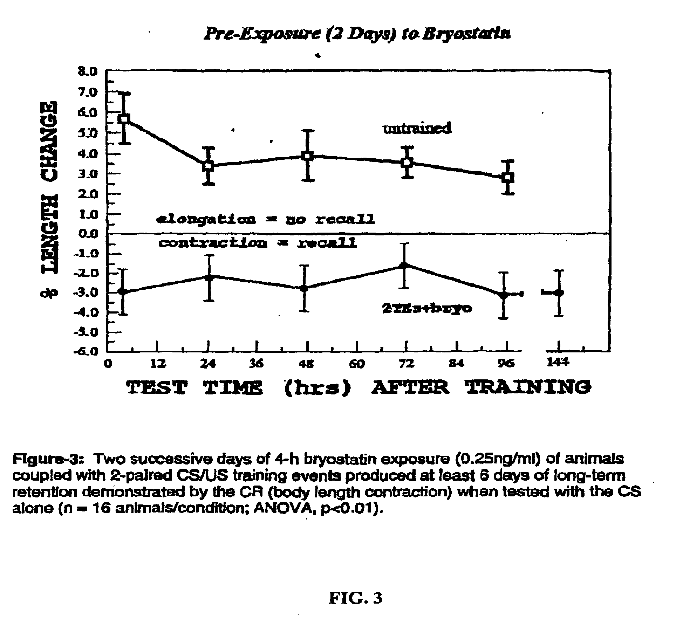

As already described, exposure to bryostatin for 4 hours during and after training enhances memory retention produced by 2 TE from 6-8 minutes to several hours. However, a 4 hour exposure to bryostatin on the day preceding training, as well as on the day of the 2 TE prolonged memory retention for more than one day after training. Two successive days of 4-h bryostatin exposure (0.25 ng/ml) of animals coupled with 2-paired CS/US training events produced at least 6 days of long-term retention demonstrated by the CR (body length contraction) when tested with the CS alone (n = 16 animals/condition; ANOVA, p<0.01) (Fig. 3).

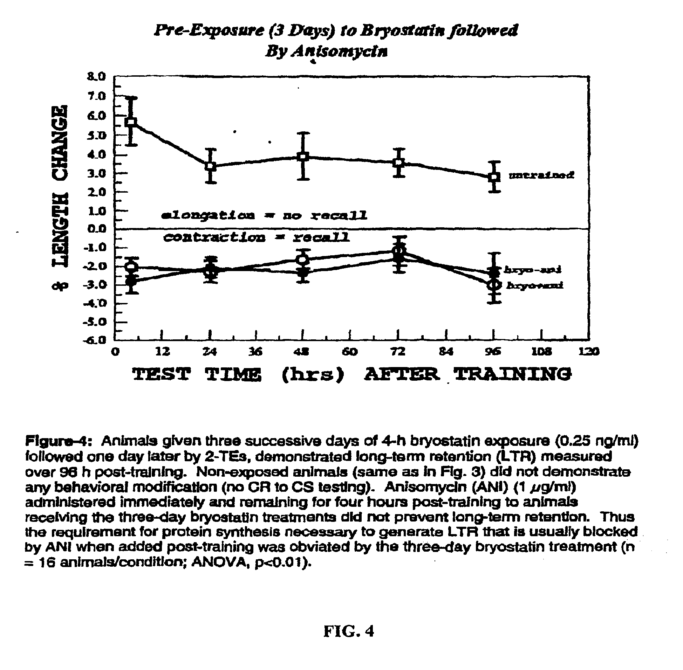

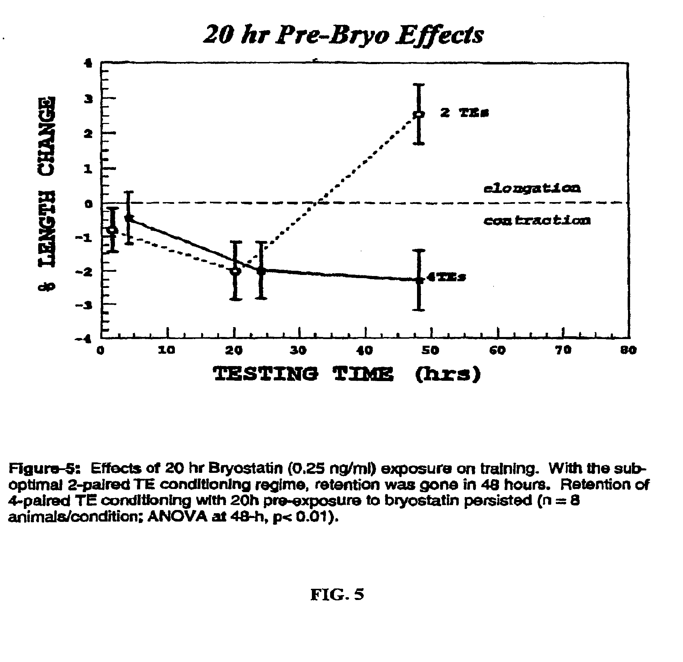

Animals given three successive days of 4-h bryostatin exposure (0.25 ng/ml) followed one day later by 2-TEs, demonstrated long-term retention (LTR) measured over 96 h post-training. Non-exposed animals (same as in Fig. 3) did not demonstrate any behavioral modification (no CR to CS testing). Anisomycin (ANI) (1 µg/ml) administered immediately and remaining for four hours post-training to animals receiving the three-day bryostatin treatments did not prevent long-term retention. Thus the requirement for protein synthesis necessary to generate LTR that is usually blocked by ANI when added post-training was obviated by the three-day bryostatin treatment (n = 16 animals/condition; ANOVA, p<0.01). A third day of exposure to the 4 hour interval of bryostatin caused a similar enhanced retention of the Pavlovian conditioned response (Fig. 4). The preceding results support the view that two successive intervals of exposure to bryostatin cause PKC activation and possibly synthesis of proteins critical for long-term memory, with a minimum of concurrent and subsequent PKC downregulation. This view was given further support by the observation that a more prolonged interval of bryostatin exposure, *i.e.* for 8 to 20 hours, followed by 2 TE (Fig. 5) was not sufficient itself to produce memory retention equivalent to that which accompanied the two 4 hour exposures on successive preceding days. In these experiments, the effects of 20 hr bryostatin (0.25 ng/ml) exposure on training was observed. With the sub-optimal 2-paired TE conditioning regime, retention was gone in 48 hours. Retention of 4-paired TE conditioning with 20h pre-exposure to bryostatin persisted (n = 8 animals/condition; ANOVA at 48-h, p< 0.01). Sufficiently prolonged bryostatin exposure (*e.g*., 8-12 hours) is known in other cell systems to cause prolonged PKC downregulation that may offset PKC activation and increase PKC synthesis.

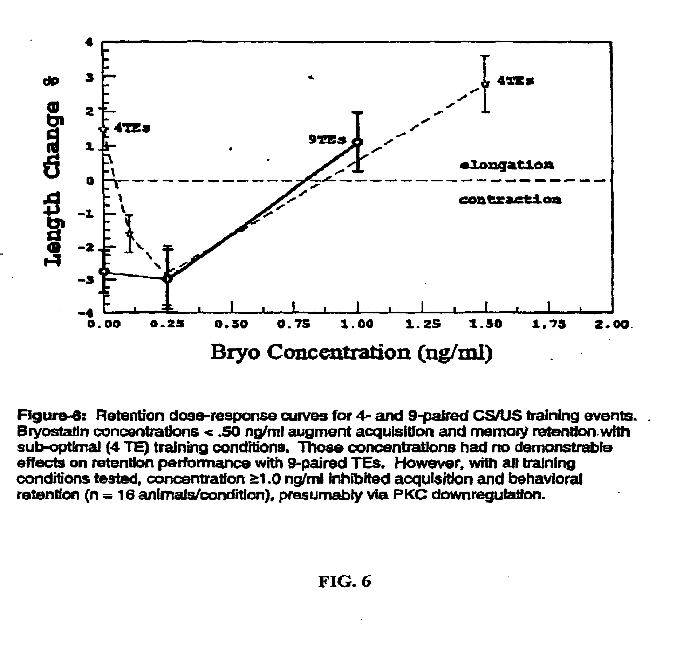

Similarly, sufficiently increased concentrations of bryostatin ultimately blocked memory retention (Fig. 6) presumably also because of PKC downregulation. Bryostatin concentrations < .50 ng/ml augment acquisition and memory retention with sub-optimal (4 TE) training conditions. Those concentrations had no demonstrable effects on retention performance with 9-paired TEs. However, with all training conditions tested, concentration ≥1.0 ng/ml inhibited acquisition and behavioral retention (n = 16 animals/condition), presumably via PKC downregulation.

### Example 8: Pre-exposure to bryostatin obviates the requirement for protein synthesis during training

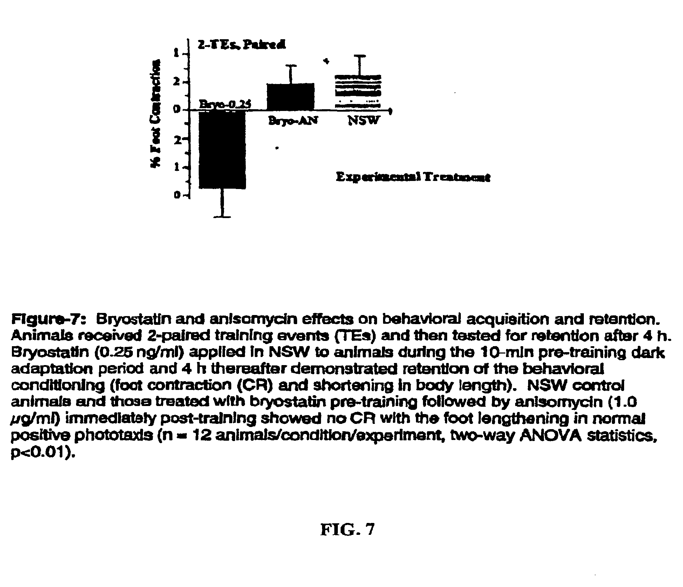

Animals received 2-paired training events (TEs) and then tested for retention after 4 h. Bryostatin (0.25 ng/ml) applied in NSW to animals during the 10-min pre-training dark adaptation period and 4 h thereafter demonstrated retention of the behavioral conditioning (foot contraction (CR) and shortening in body length). NSW control animals and those treated with bryostatin pre-training followed by anisomycin (1.0 µg/ml) immediately post-training showed no CR with the foot lengthening in normal positive phototaxis (n = 12 animals/condition/experiment, two-way ANOVA statistics, p<0.01). A single 4 hour exposure to bryostatin together with 2 TE produced long-term memory retention lasting hours that was entirely eliminated when anisomycin was present along with the bryostatin (Fig. 7). Similar blocking effects of anisomycin were also observed with 6 TE plus bryostatin. Repeated brief exposures to bryostatin, however, increase the net synthesis of PKC, calexcitin, and other memory proteins and thus eliminate the requirement for new synthesis during and after Pavlovian conditioning-if PKC downregulation were sufficiently minimized. Protein synthesis was blocked for 4 hours with anisomycin immediately after 2 TE of animals that on each of 3 preceding days had been first exposed to 4 hours of bryostatin. In this case, anisomycin-induced blockade of protein synthesis did not prevent memory retention that lasted many days (Fig. 4). By contrast, the same 4 hour anisomycin treatment eliminated all memory retention produced by 9 TE, a training regimen ordinarily followed by 1-2 weeks of memory retention (27). Finally, if 2 TE were given one day after three successive days of 4 hour exposures to bryostatin that was accompanied each time by anisomycin, long-term memory was eliminated.

### Example 9: Pre-exposure to proteasome inhibition enhances bryostatin effects on memory

Another means of enhancing and prolonging *de novo* synthesis of PKC and other memory-related proteins is provided by blocking pathways involved in protein degradation. One of these, the ubiquitin-proteasome pathway (28-30), is known to be a major route for degradation of the α-isozyme of PKC. Degradation of PKC-α has been previously shown to be largely prevented by 20µM-5QµM of the proteasome inhibitor, Lactacysteine.

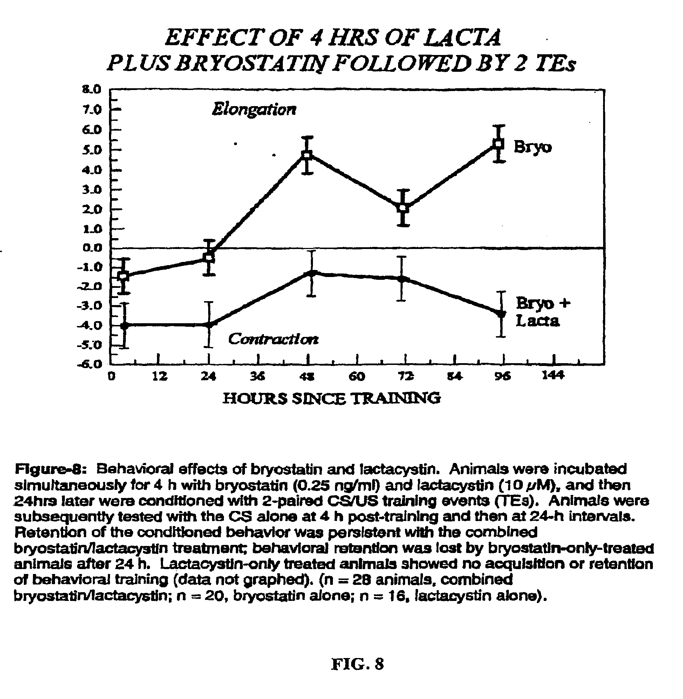

Animals were incubated simultaneously for 4 h with bryostatin (0.25 ng/ml) and lactacysteine (10 µM), and then 24 hrs later were conditioned with 2-paired CS/US training events (TEs). Animals were subsequently tested with the CS alone at 4 h post-training and then at 24-h intervals. Retention of the conditioned behavior was persistent with the combined bryostatin/lactacysteine treatment; behavioral retention was lost by bryostatin-only-treated animals after 24 h. Lactacysteine-only treated animals showed no acquisition or retention of behavioral training (data not graphed). (n = 28 animals, combined bryostatin/lactacysteine; n = 20, bryostatin alone; n = 16, lactacysteine alone). Lactacysteine, in this case, transformed the short-term memory produced by the single bryostatin exposure (followed by 2 TE) to long-term memory lasting many days (Fig. 8).

### Example 10: Calexcitin-Immunostaining due to PKC activation

Recently we showed that an immunostaining label of calexcitin increased within single identified Type B cells during acquisition and retention of *Hermissenda* conditioning (20). Many previous findings have implicated a low molecular weight calcium and GTP-binding protein, calexcitin, as a substrate for PKC isozymes during *Hermissenda* conditioning (19). Calexcitin, now completely sequenced in some animal species, and shown to have significant homology with similar proteins in other species (31), undergoes changes of phosphorylation during and after *Hermissenda* Pavlovian conditioning. It is also a high affinity substrate for the alpha-isozyme of PKC and a low affinity substrate for β and gamma (19).

Micrographs (A, B) depict representative tissue sections from *Hermissenda* eyes that were immunolabeled with the calexcitin polyclonal antibody, 25U2. Positive calexcitin immunostaining occurred in B-cell photoreceptors (*B-Cell) of animals that experienced paired CS/UCS associative conditioning with or without prior administration of bryostatin (B). Random presentations of the two stimuli (training events, TEs) did not produce behavioral modifications nor a rise in calexcitin above normal background levels (A); basement membrane and lens staining are artifact associated with using vertebrate polyclonal antibodies. Differences in staining intensities were measured and recorded as gray-scale intensities (0-256; B-cell cytoplasm minus tissue background). Graph (C) displays intensity measures for *Hermisssenda* conditioned with 9-random TEs (left bar) and animals treated with two exposures on successive days to the PKC agonist, bryostatin (0.25 ng/ml), and then associatively conditioned with 2-paired TEs. Activation of PKC from two exposures of bryostatin coupled with 2 TEs significantly increased calexcitin to levels associated with 9-paired TEs and consolidated (long-term) memory (n = 4-8 animals/condition/replicate; *t*-test comparison, p<0.01).

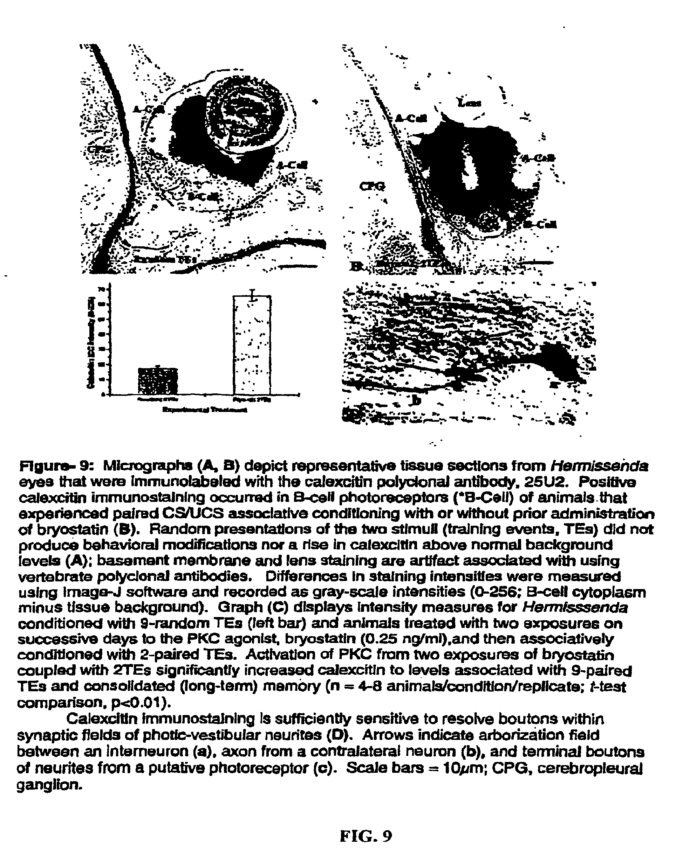

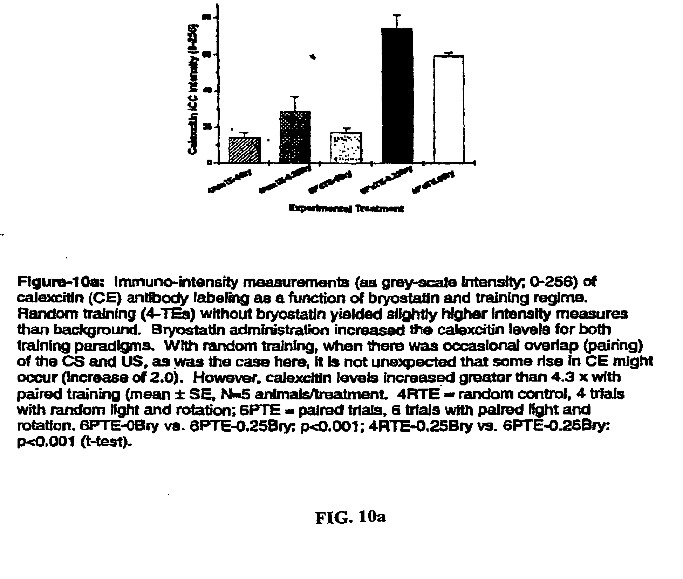

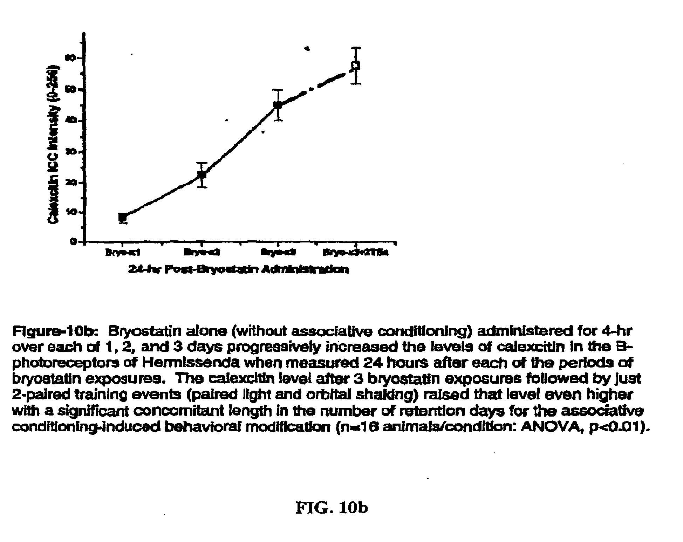

Calexcitin immunostaining is sufficiently sensitive to resolve boutons within synaptic fields of photic-vestibular neurites (D). Arrows indicate arborization field between an interneuron (a), axon from a contralateral neuron (b), and terminal boutons of neurites from a putative photoreceptor (c). Scale bars = 10µm; CPG, cerebropleural ganglion (Fig. 9, 10).

This conditioning-induced calexcitin label increase represents an increase in the actual amount of the protein since the immunostaining antibody reacts with both the phosphorylated and unphosphorylated forms of the protein. PKC, previously shown to translocate within the same individual Type B cells, apparently caused the conditioning-induced increase in the calexcitin label since the specific PKC-blocker, Ro-32, prevented both learning and learning-specific calexcitin increases in the Type B cell (see above). Naïve and/or randomized control training protocols produced a small fraction of the training-induced calexcitin (CE) immunostaining (Fig 9).

Random training (4-TEs) without bryostatin yielded slightly higher intensity measures than background. Bryostatin administration increased the calexcitin levels for both training paradigms. With random training, when there was occasional overlap (pairing) of the CS and US, as was the case here, it is not unexpected that some rise in CE might occur (increase of 2.0). However, calexcitin levels increased greater than 4.3 x with paired training (mean ± SE, N=5 animals/treatment. 4RTE = random control, 4 trials with random light and rotation; 6PTE = paired trials, 6 trials with paired light and rotation. 6PTE-OBry vs. 6PTE-0.25Bry: p<0.001; 4RTE-0.25Bry vs. 6PTE-0.25Bry; p<0.001 (t-test). When sub-optimal training events (4-6 TE) were used, the CE immunostaining (Fig 10A) reached an intermediate level of elevation. These sub-optimal regimes were insufficient to produce memory retention lasting longer than 24 hours. As described earlier, bryostatin administered during training with 6 TE induced long-term memory retention (> 1 week). Furthermore, bryostatin plus 6 TE induced CE immunostaining comparable to that observed after 9 TE.

Bryostatin in low doses (0.1-0.25ng/ml) markedly enhanced memory after 2, 4, or 6 training trials. Pavlovian conditioning with 6 TE produced memory lasting many days with bryostatin, but lasting only hours without bryostatin. This memory enhancement was blocked by anisomycin or the PKC inhibitor, Ro-32. It is important to note that CE immunostaining was greatly reduced 24 hours after 9 TE even though the memory persisted for more than 1 week thereafter. More persistent CE immunostaining resulted, however, from repeated bryostatin exposures on days preceding minimal training (2 TE).

Bryostatin alone (without associative conditioning) administered for 4-hr over each of 1, 2, and 3 days progressively increased the levels of calexcitin in the B-photoreceptors of *Hermissenda* when measured 24 hours after each of the periods of bryostatin exposures. Twenty-four hours after 1 bryostatin exposure for four hours, CE immunostaining was not elevated (Fig. 10B). Twenty-four hours after 2 bryostatin exposures, 1 on each of two successive days showed greater residual CE immunostaining. The calexcitin level after 3 bryostatin exposures followed by just 2-paired training events (paired light and orbital shaking) raised that level even higher with a significant concomitant length in the number of retention days for the associative conditioning-induced behavioral modification (n=16 animals/condition: ANOVA, p<0.01). With 2 TE on the subsequent day after these three exposures, CE immunostaining 24 hours later approached the levels previously observed immediately following 9 TE (Fig. 10B). Thus, CE immunostaining following these three days of 4 hour bryostatin exposure followed by minimal training (2 TE) showed a greater persistence than did the training trials alone. This persistence of newly synthesized calexcitin is consistent with the biochemical observations indicating enhanced protein synthesis induced by bryostatin.

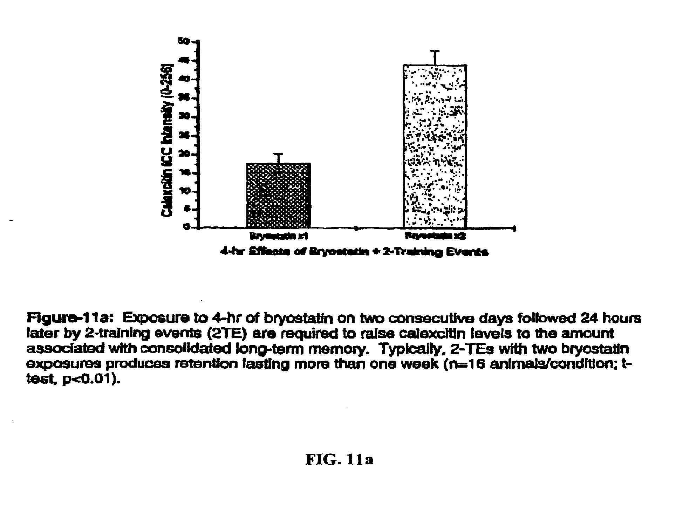

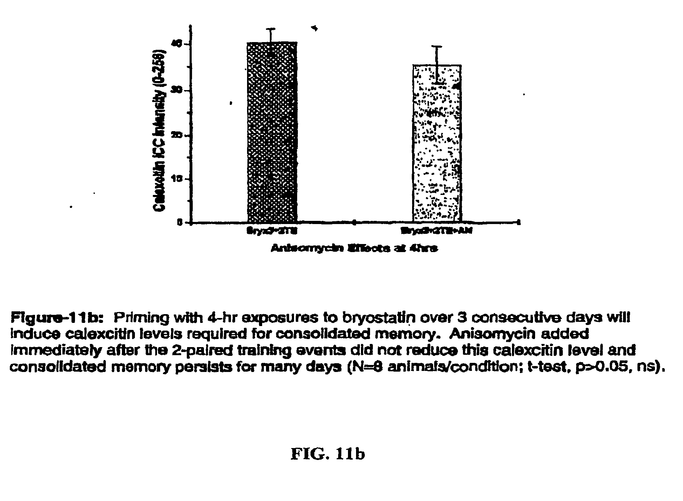

Exposure to 4-hr of bryostatin on two consecutive days followed 24 hours later by 2-training events (2 TE) are required to raise calexcitin levels to the amount associated with consolidated long-term memory. Typically, 2-TEs with two bryostatin exposures produces retention lasting more than one week (n=16 animals/condition; t-test, p<0.01). Priming with 4-hr exposures to bryostatin over 3 consecutive days will induce calexcitin levels required for consolidated memory. Anisomycin added immediately after the 2-paired training events did not reduce this calexcitin level and consolidated memory persists for many days (N=8 animals/condition; t-test, p>0.05, ns). (Figs. 11 A, B).

It is noteworthy that the Ro-32 inhibition of PKC immediately after bryostatin plus training did not prevent long-term memory induction, while this inhibition *during* the training plus bryostatin did prevent memory consolidation. In contrast, anisomycin *during training* with and without bryostatin did not prevent long-term memory, while anisomycin after training with and without bryostatin completely blocked memory formation. Therefore, PKC activation during training is followed by protein synthesis required for long-term memory. Thus, once PKC activation is induced to sufficient levels, the required protein synthesis is an inevitable consequence. Consistently, bryostatin-induced PKC activation on days prior to training is sufficient, with minimal training trials, to cause long-term memory. Furthermore, this latter long-term memory does not require protein synthesis following the training (and PKC activation on preceding days). Again, prior PKC activation was sufficient to produce that protein synthesis necessary for subsequent long-term memory formation. One of those proteins whose synthesis is induced by bryostatin-induced PKC activation as well as conditioning trials is cafexcitin-as demonstrated by the immunostaining labeling. The other protein is PKC itself.

### Example 11: Effect of Bryostatin on PKC Activity

Bryostatin is known to transiently activate PKC by increasing PKC association with the cellular membrane fraction. A variety of associative memory paradigms have also been demonstrated to cause increased PKC association with neuronal membranes. We tested, therefore, the possibility that repeated exposures of *Hermissenda* to bryostatin (*i.e*., 4 hour exposures, exactly as with the training protocols) might also induce prolonged PKC activation.

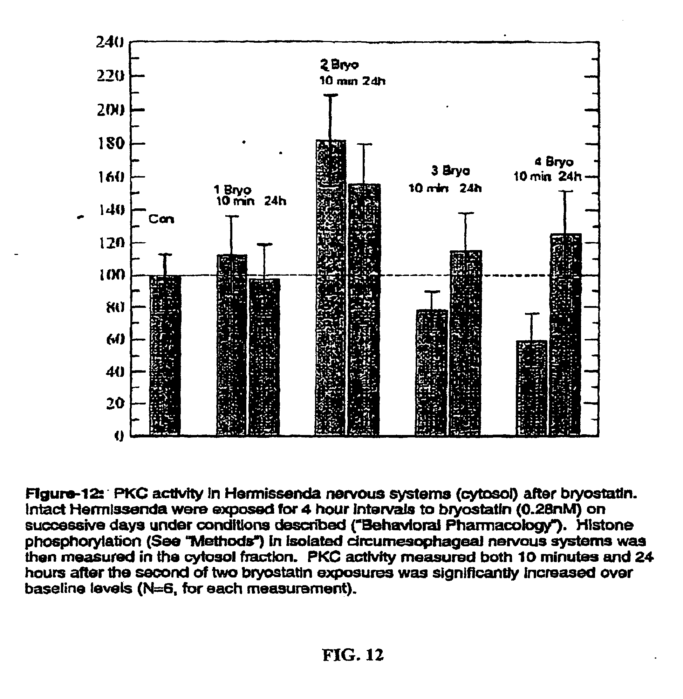

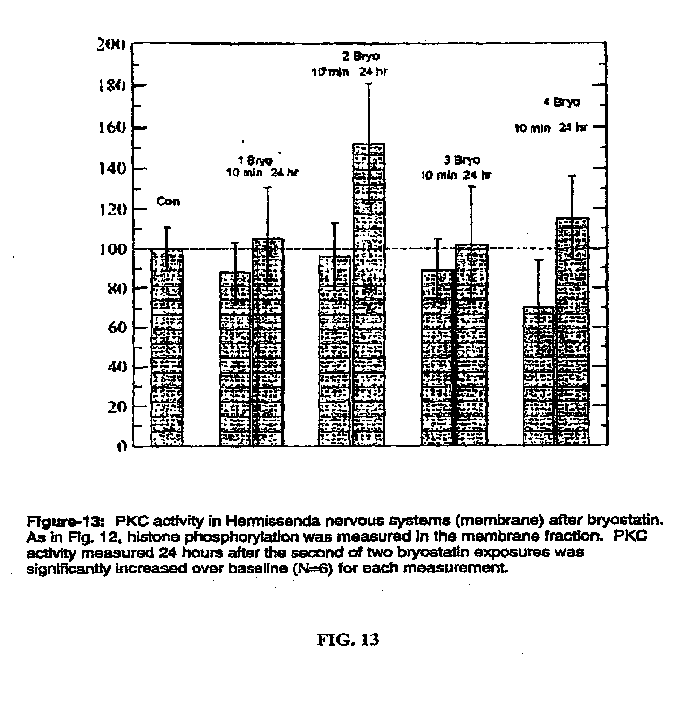

Intact *Hermissenda* were exposed for 4 hour intervals to bryostatin (0.28nM) on successive days under conditions described ("Behavioral Pharmacology"). Histone phosphorylation (See "Methods") in isolated circumesophageal nervous systems was then measured in the cytosol fraction. PKC activity measured both 10 minutes and 24 hours after the second of two bryostatin exposures was significantly increased over baseline levels (N=6, for each measurement). (Fig. 12, 13). Thus, the quantity of PKC in both fractions was apparently increased, but not the ratio of the PKC in the membrane to that in the cytosolic fraction. These results demonstrate that the bryostatin pre-exposure causes an effect on PKC somewhat different from learning itself. After an initial activation (via translocation), this bryostatin effect is most likely due to increased synthesis of PKC, consistent with the increased levels of calexcitin induced by bryostatin, but not directly correlated with repeated bryostatin exposure.

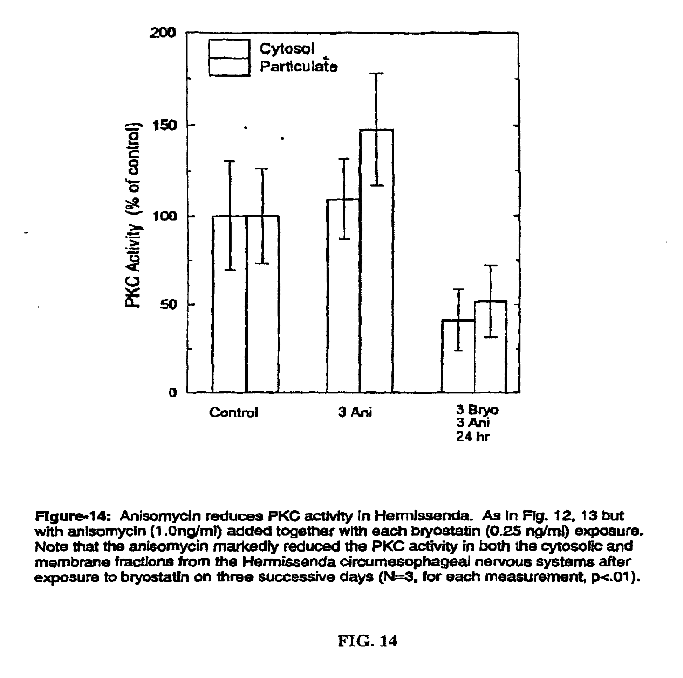

As in Fig. 12, 13 but with anisomycin (1.0 ng/ml) added together with each bryostatin (0.25 ng/ml) exposure. Note that the anisomycin markedly reduced the PKC activity in both the cytosolic and membrane fractions from the *Hermissenda* circumesophageal nervous systems after exposure to bryostatin on three successive days (N=3, for each measurement, p<-01) (Fig. 14).

To further examine biochemical consequences of repeated exposures to bryostatin, rat hippocampal neurons were studied after they had been immortalized by retroviral transduction of temperature sensitive tsA5CSV40 large T antigen (25). These differentiate to have a neuronal phenotype when induced by basic fibroblast growth factor in N2 medium (26) and express a normal complement of neuronal proteins, including PKC.

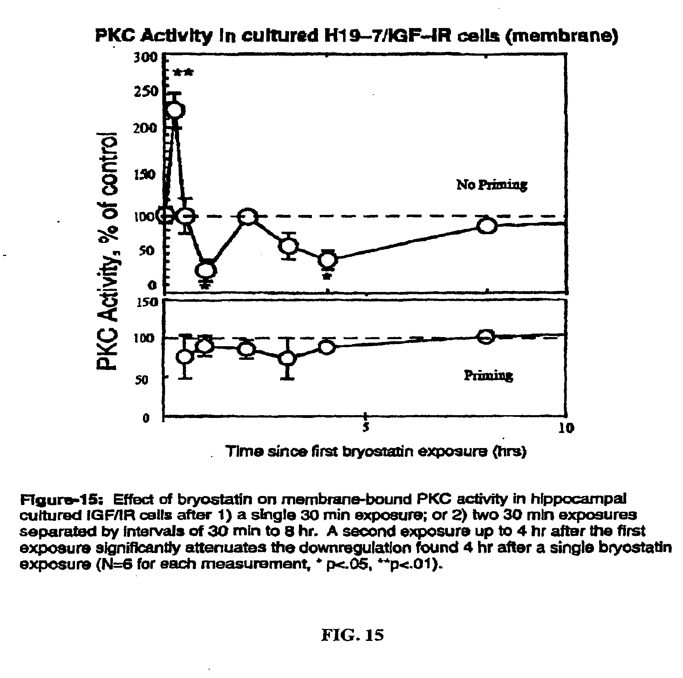

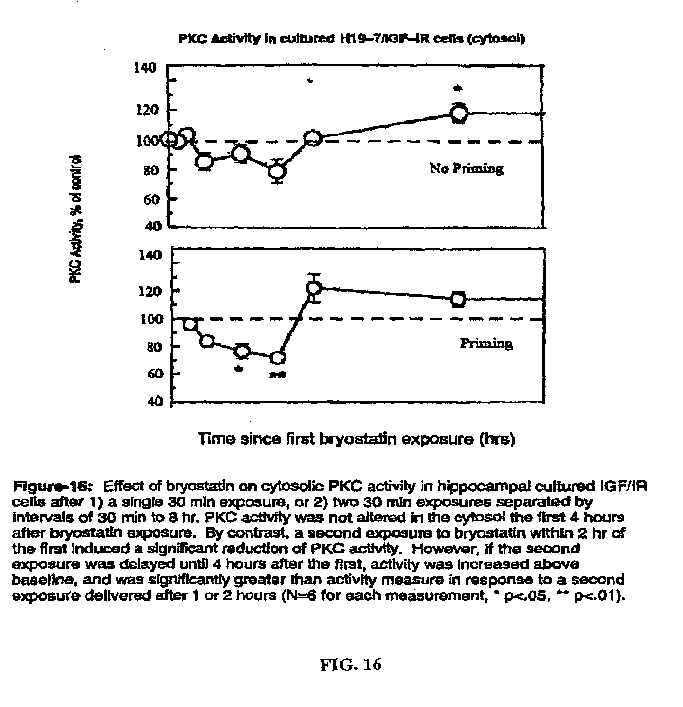

Exposure of cultured hippocampal neurons to a single activating dose of bryostatin (0.28 nM) for 30 minutes produced a brief translocation of PKC from the cytosol to the particulate fraction (approx 60%) followed by a prolonged downregulation (Fig. 15). Both the initial PKC activation and subsequent downregulation have been previously described and were confirmed by measurement of PKC activity in membrane and cytosol. Exposing the cultured hippocampal neurons to one 30-minute period of bryostatin, followed by a second 30-minute exposure, at intervals ranging from 30 minutes to 8 hours, caused the membrane-bound PKC to rebound more quickly. Thus, a second exposure after a 2- to 4-hour delay eliminated the significant downregulation that a single bryostatin exposure produced (Fig. 16). In the cytoplasmic fraction, no significant alteration of PKC activity was detected within the first 4 hours after bryostatin exposure. In contrast, if cells were exposed to bryostatin twice within a 2-hour period, there was a significant reduction of PKC activity in response to the second exposure. However, if the second exposure was delayed until 4 hours after the first, activity was increased above baseline, to a degree that was significantly greater compared with a second exposure delivered after 2 hours or less (Fig. 16).

These results are consistent with the interpretation that the initial bryostatin activation of PKC followed by downregulation (28-30) leads to increased synthesis (via *de novo* protein synthesis) of PKC isozymes (as well as calexcitin, described earlier). In fact, we found here that a single 30-minute exposure to 0.28 nM bryostatin increased overall protein synthesis (Fig. 17), measured by incorporation of ³⁵S-methionine in the last ½ hour before collecting the neurons, by 20% within 24 h, increasing to 60% by 79 hours after the bryostatin exposure. This prolonged and profound increase of protein synthesis induced by bryostatin was partially blocked when the PKC inhibitor Ro-32 was also present (Fig. 17).

Abundant observations indicate that sufficient bryostatin-induced PKC activation leads, inevitably, to progressive PKC inactivation and subsequent downregulation. Sufficient doses of bryostatin (greater than 1.0 ng/ml) actually inhibited Pavlovian conditioning. This was most likely due to PKC downregulation that characterized the behavioral results with high bryostatin concentrations. PKC activation induced by bryostatin has been shown to be downregulated by two distinct pathways. One that is also induced by phorbol ester involves ubiquitination and subsequent proteolytic degradation through the proteasome pathway. The second mechanism of downregulation, not induced by phorbol ester, involves movement through caveolar compartments and degradation mediated by phosphatase PP1 and PP2A. With sufficient concentrations and/or durations of PKC activators, the PKC degrading pathways create a deficit of PKC that stimulates *de novo* synthesis of PKC, PKC synthesis cannot compensate for inactivation and downregulation, thereby causing depletion of available PKC of 95% or more.

### Example 12: Effects of Bryostatin on Learning and Retention of Memory

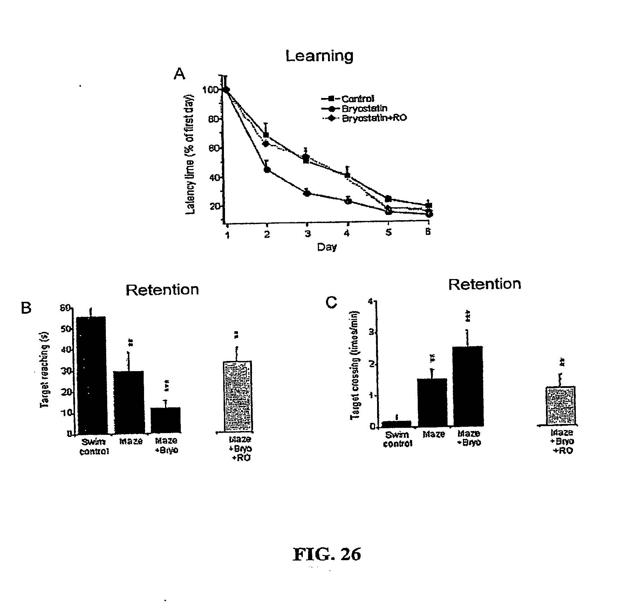

The effects of bryostatin on learning and memory were examined using the rat spatial maze model. Bryostatin (NCI, 10µg/kg body wt.) was intraperitoneally injected 20 min before water maze training on day 1, 3, and 5. RO-31-8220 (Sigma, 500µg/kg body wt.) was injected into a tail vein 10 min before bryostatin injection. The results are shown in Figure 26. Asterisks are significantly different from swim controls (**, p<.01; **, p<.001). In probe tests, Maze+Bryo is significantly different from Maze and from Maze+Bryo+RO (p<.05).

In Panel A of Figure 26, the latency for rats to reach the platform, is greatly reduced (i.e., learning is facilitated) in bryostatin-treated animals vs. controls, but not in the presence of the PKC-α inhibitor, RO-31-8220. In Panel B, the time to reach the target quadrant is reduced (i.e., memory retention is enhanced) on retention day 1, 24 hours after all training, for bryostatin-treated animals vs. controls, but not in the presence of RO-31-8220. In Panel C, the number of target crossings 1 day after all training is similarly enhanced (i.e., memory retention is enhanced) on retention Day 1 for the bryostatin treated mice.

### Example 13: Effects of Bryostatin on Dendritic Spines of Rats Trained In A Spatial Maze Task

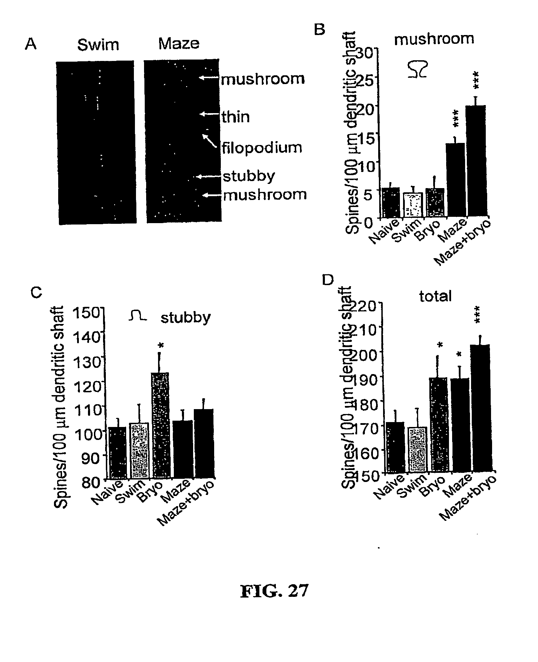

Figure 27 shows the effects of bryostatin on dendritic spine formation in rats of trained in a water maze. On retention day 2, confocal microscopy and Dil staining were used to study filopodia and dendritic spines: mushroom, thin and stubby spines (Panel A). Water maze training increased the number of mushroom spines; this effect was enhanced by bryostatin (Panel B). Bryostatin alone (without training) augmented the number of stubby spines (p<.01) (Panel C). Under all conditions, no changes in filopodia or thin spines were seen (not shown). The total numbers of filopodia and (all shape) spines in rats treated with only bryostatin and those only receiving training were similarly increased (Panel D). This increase was enhanced when water maze rats were also treated with bryostatin. (p<.05). Asterisks indicate significantly differences from naive controls (*, p<.05; **, p<.001).

### Example 14: Effects of Bryostatin On Mushroom Spines

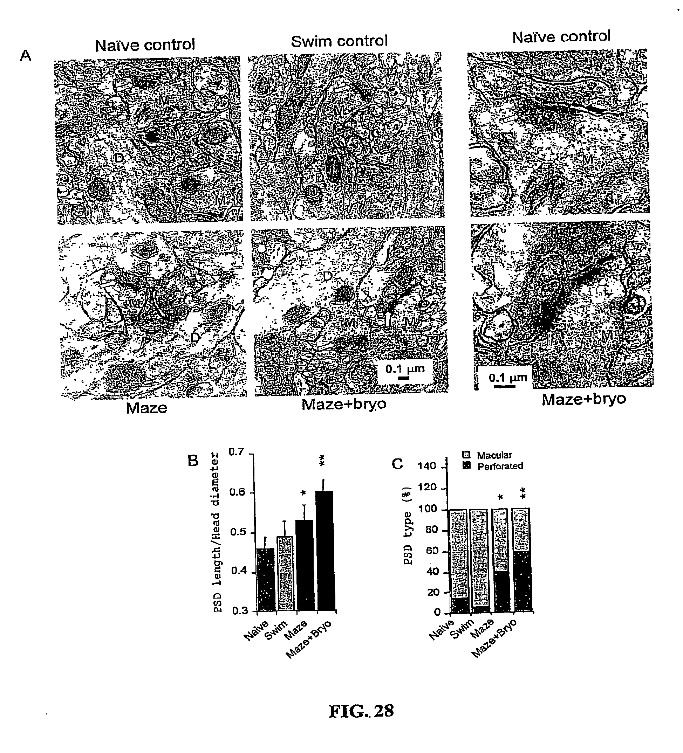

Figure 28 depicts electron micrographs of the changes observed in mushroom spines (M) and postsynaptic densities (PSD; yellow arrows); red arrow=presynaptic membrane; D=dendrite after bryostatin treatment and training (Panel A). Maze+bryo panel is perforated PSD (large PSD with hole in the center), whereas those in other panels are macular type (small PSD without hole). Water maze training with or without bryostatin enlarged the averaged size of PSD (Panel B), due to the increased number of large mushroom spines with perforated PSD (Panel C). Asterisks are significantly different from naive controls (*, p<.05; **, p<.001).

### Example 15: Different effects of Bryostatin on Pre- and Postsynaptic Structures

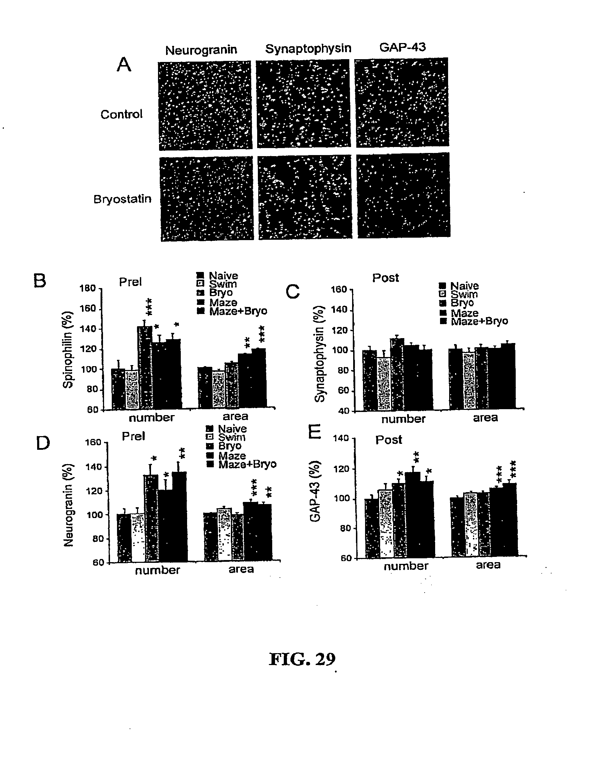

Water maze training increased the numbers of the dendritic spine marker spinophilin (Figure 29; Panel B), postsynaptic membrane marker neurogranin (Figure 29; Panels A and D), and presynaptic marker GAP-43 (Panels A and E), but not the axon bouton marker synaptophysin (Figure 29; Panels A and B), as determined by quantitative confocal immunohistochemistry. These results show that a new spine forms a synapse with a preexisting axon bouton that already made a synapse with preexisting spine(s). The number of presynaptic markers were also increased in rats receiving bryostatin alone. Water maze training, with or without bryostatin treatment, increased the sizes of pre- and postsynaptic membranes, confirming that water maze training selectively increases mushroom spines with large PSD. Asterisks indicate significantly differences from naive controls (*, p<.05; **, p<.01; ***, p<.001).

### Example 16: Mechanism of Increased Spine Density by PKC Activation

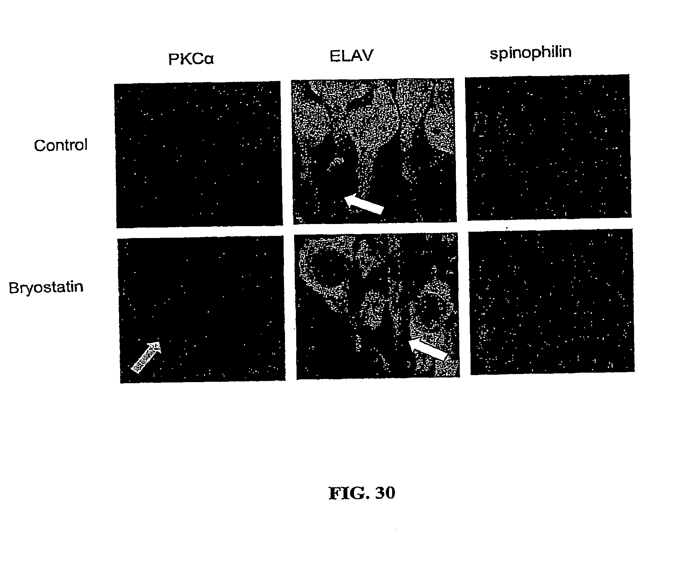

The mechanism of increased spine density by PKC activation is shown in Figure 30. Acute hippocampal slices were continuously incubated with 0.1 nM bryostatin and then processed for quantitative confocal immunohistochemistry. Bryostatin stimulates translocation to the plasma membrane (yellow arrow) and activation of PKCα and the nuclear export of PKC-dependent ELAV, mRNA-stabilizing proteins, to the cytoplasm in the cell bodies and proximal dendrites of CA1 neurons (white arrows). Bryostatin also increased the number of dendritic spines, determined by the spine marker spinophilin.

### Example 17: Mechanism of Increased Spine Density by PKC Activation

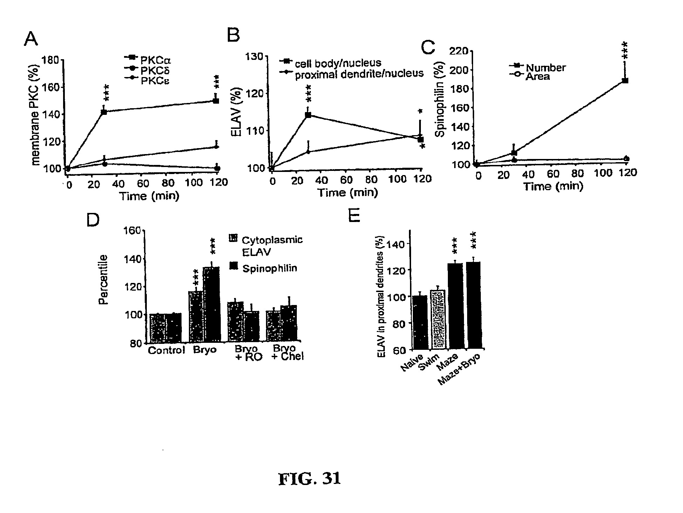

The mechanism of increased spine density by PKC activation is shown in Figure 31. In hippocampal slices, bryostatin selectively activated PKCα, but not PKCδ and PKCε (Panel A). When ELAV significantly transported to dendrites at 120-min incubation with bryostatin (Panel B), the number of dendritic spines was augmented (Panel C). These effects were suppressed by the PKC blocker RO-31-8220 or chelerythrine (Panel D). Increased spine density was also inhibited by the protein synthesis blocker (not shown). Altogether, these suggest that bryostatin stimulates PKCα-activated ELAV proteins, leading to an inhibition of mRNA degradation and enhancement of protein synthesis that is important for spine formation. At day 2 after the probe test and 6-days water maze training, ELAV was still elevated in dendrites (Panel E), suggesting that water maze increases mushroom spine density by PKC/ELAV/protein synthesis cascade. However, sustained increase in ELAV in dendrites is not different after spatial learning with and without bryostatin, suggesting that PKC/ELAV/protein synthesis is not the only pathway for mushroom spine formation. Asterisks indicate significantly differences from naive controls (*, p<.05; **, p<.01; ***, p<.001).

In the following clauses, preferred embodiments of the invention are described:
1. A method comprising the step of contacting a PKC activator with a protein kinase C (PKC) to stimulate cellular or neuronal growth.
2. The method of clause 1, wherein the contacting a PKC activator with a protein kinase C (PKC) stimulates dendritic growth.
3. The method of clause 1, wherein the contacting a PKC activator with a protein kinase C (PKC) stimulates dendritic spine formation.
4. The method of clause 1, wherein the contacting a PKC activator with a protein kinase C (PKC) stimulates dendritic spine density.
5. The method of clause 1, wherein the contacting a PKC activator with a protein kinase C (PKC) stimulates ELAV translocation to proximal dendrites.
6. The method of clause 1, wherein said PKC activator is a macrocyclic lactone.
7. The method of clause 1, wherein the PKC activator is a benzolactam.
8. The method of clause 1, wherein the PKC activator is a pyrrolidinone.
9. The method of clause 6, wherein the macrocyclic lactone is a bryostatin.
10. The method of clause 9, wherein the bryostatin is bryostatin-1, -2, -3, -4, -5, -6, -7, -8, -9, -10, -11, -12, -13, -14, -15, -16, -17, or -18.
11. The method of clause 9, wherein the bryostatin is bryostatin-1.
12. The method of clause 6, wherein the macrocyclic lactone is a neristatin.
13. The method of clause 12, wherein the neristatin is neristatin- 1.
14. The method of clause 1, wherein said contact activates PKC.
15. The method of clause 1, wherein said contact increases the amount of PKC.
16. The method of clause 1, wherein said contact increases the synthesis of PKC.
17. The method of clause 14, wherein the PKC is PKC[alpha].
18. The method of clause 155 wherein the PKC is PKC[alpha].
19. The method of clause 16, wherein the PKC is PKC[alpha].
20. The method of clause 1, wherein said contact increases the amount of calexcitin.
21. The method of clause 1, wherein said contact does not result in substantial subsequent downregulation of PKC.
22. The method of clause 1, wherein the contacting of the PKC activator with the PKC is repeated.
23. The method of clause 22, wherein the contacting of the PKC activator with the PKC is repeated at regular intervals.
24. The method of clause 23, wherein the interval is between one week to one month, one day and one week, or less than one hour and 24 hours.
25. The method of clause 24, wherein the interval is between one week and one month.
26. The method of clause 24, wherein the interval is between one day and one week.
27. The method of clause 24, wherein the interval is between less than one hour and 24 hours.
28. The method of clause 13 wherein the contacting of the PKC activator with the PKC is maintained for a fixed duration.
29. The method of clause 28, wherein the fixed duration is less than 24 hours.
30. The method of clause 28, wherein the fixed duration is less than 12 hours.
31. The method of clause 28, wherein the fixed duration is less than 6 hours.
32. The method of clause 28, wherein the fixed duration is less than 4 hours.
33. The method of clause 28, wherein the fixed duration is less than 2 hours.
34. The method of clause 28, wherein the fixed duration is between about 2 and about 6 hours.
35. The method of clause 28, wherein the fixed duration is about 4 hours.
36. The method of clause 28, wherein said duration of said contact is between about 1 and about 12 hours.
37. The method of clause 22, wherein said contact is repeated for a period greater than one day.
38. The method of clause 22, wherein said contact is repeated for a period between one day and one month.
39. The method of clause 22, wherein said contact is repeated for a period between one day and one week.
40. The method of clause 22, wherein said contact is repeated for a period between one week and one month.
41. The method of clause 22, wherein said contact is repeated for a period between one month and six months.
42. The method of clause 22, wherein said contact is repeated for a period of one month.
43. The method of clause 22, wherein said contact is repeated for a period greater than one month.
44. A method comprising the step of contacting a PKC activator with a protein kinase C (PKC) to stimulate the synthesis of proteins sufficient to consolidate long term memory.
45. The method of clause 44, wherein said PKC activator is a macrocyclic lactone.
46. The method of clause 44, wherein the PKC activator is a benzolactam.
47. The method of clause 44, wherein the PKC activator is a pyrrolidinone.
48. The method of clause 45, wherein the macrocyclic lactone is a bryostatin.
49. The method of clause 48, wherein the bryostatin is bryostatin-1, -2, -3, -4, -5, -6, -7, - 8, -9, -10, - 11, -12, -13, -14, -15, -16, -17, or -18.
50. The method of clause 48, wherein the bryostatin is bryostatin-1.
51. The method of clause 45, wherein the macrocyclic lactone is a neristatin.
52. The composition of clause 51, wherein the neristatin is neristatin- 1.
53. The method of clause 44, wherein said contact activates PKC.
54. The method of clause 44, wherein said contact increases the amount of PKC.
55. The method of clause 44, wherein said contact increases the synthesis of PKC.)
56. The method of clause 44, wherein said contact increases the amount of calexcitin.
57. The method of clause 44, wherein said contact does not result in substantial subsequent downregulation of PKC.
58. The method of clause 44, wherein the contacting of the PKC activator with the PKC is repeated.
59. The method of clause 58, wherein the contacting of the PKC activator with the PKC is repeated at regular intervals.
60. The method of clause 59, wherein the interval is between one week to one month, one day and one week, or less than one hour and 24 hours.
61. The method of clause 60, wherein the interval is between one week and one month.
62. The method of clause 60, wherein the interval is between one day and one week.
63. The method of clause 60, wherein the interval is between less than one hour and 24 hours.
64. The method of clause 44, wherein the contacting of the PKC activator with the PKC is maintained for a fixed duration.
65. The method of clause 64, wherein the fixed duration is less than 24 hours.
66. The method of clause 64, wherein the fixed duration is less than 12 hours.
67. The method of clause 64, wherein the fixed duration is less than 6 hours.
68. The method of clause 64, wherein the fixed duration is less than 4 hours.
69. The method of clause 64, wherein the fixed duration is less than 2 hours.
70. The method of clause 64, wherein the fixed duration is between about 2 and about 6 hours.
71. The method of clause 64., wherein the fixed duration is about 4 hours.
72. The method of clause 64, wherein said duration of said contact is between about 1 and about 12 hours.
73. The method of clause 58, wherein said contact is repeated for a period greater than one day.
74. The method of clause 58, wherein said contact is repeated for a period between one day and one month.
75. The method of clause 58, wherein said contact is repeated for a period between one day and one week.
76. The method of clause 58, wherein said contact is repeated for a period between one week and one month.
77. The method of clause 58, wherein said contact is repeated for a period between one month and six months.
78. The method of clause 58, wherein said contact is repeated for a period of one month.
79. The method of clause 58, wherein said contact is repeated for a period greater than one month.
80. A method comprising the step of contacting a PKC activator with a protein kinase C (PKC) to downregulate PKC.
81. The method of clause 80, wherein said PKC activator is a macrocyclic lactone.
82. The method of clause 80, wherein the PKC activator is a benzolactam.
83. The method of clause 80, wherein the PKC activator is a pyrrolidinone.
84. The method of clause 81, wherein the macrocyclic lactone is a bryostatin.
85. The method of clause 84, wherein the bryostatin is bryostatin-1, -2, -3, -4, -5, -6, -7, - 8, -9, -10, - 11, -12, -13, -14, -15, -16, -17, or -18.
86. The method of clause 85, wherein the bryostatin is bryostatin-1.
87. The method of clause 81, wherein the macrocyclic lactone is a neristatin.
88. The method of clause 81, wherein the neristatin is neristatin-1.
89. The method of clause 80, wherein said contact produces downregulation of PKC.
90. The method of clause 89, wherein said contact produces substantial downregulation of PKC.
91. The method of clause 80, wherein said contact does not stimulate the synthesis of PKC.
92. The method of clause 91, wherein said contact does not substantially stimulate the synthesis of PKC.
93. The method of clause 80, wherein said contact decreases the amount of PKC.
94. The method of clause 93, wherein said contact substantially decreases the amount of PKC.
95. The method of clause 80, wherein said contact does not stimulate the synthesis of calexcitin.
96. The method of clause 93, wherein said contact does not stimulate the synthesis of calexitin.
97. The method of clause 80, wherein the contacting of the PKC activator with the PKC is for a sustained period.
98. The method of clause 97, wherein the sustained period is between less than one hour and 24 hours.
99. The method of clause 97, wherein the sustained period is between one day and one week.
100. The method of clause 97, wherein the sustained period is between one week and one month.
101. The method of clause 97, wherein the sustained period is between less than one hour and 12 hours.
102. The method of clause 97, wherein the sustained period is between less than one hour and 8 hours.
103. The method of clause 97, wherein the sustained period is between less than one hour and 4 hours.
104. The method of clause 97, wherein the sustained period is about 4 hours.
105. The method of clause 80, wherein said contact produces sustained downregulation of PKC.
106. The method of clause 44, further comprising the step of inhibiting degradation of protein kinase C (PKC).
107. The method of clause 106, wherein said degradation is through ubiquitination.
108. The method of clause 107, wherein said degradation is inhibited by lactacysteine.
109. The method of clause 44, wherein the PKC is human.
110. The method of clause 44, wherein the PKC activator is provided in the form of a pharmaceutical composition comprising the PKC activator and a pharmaceutically acceptable carrier.
111. The method of clause 110, wherein the pharmaceutical composition further comprises a PKC inhibitor.
112. The method of clause 111, wherein the PKC inhibitor inhibits PKC in peripheral tissues.
113. The method of clause 111, wherein the PKC inhibitor selectively inhibits PKC in peripheral tissues.
114. The method of clause 111, wherein the PKC inhibitor is a compound that reduces myalgia associated with the administration of a PKC to a subjects.
115. The method of clause 111, wherein the PKC inhibitor is a compound that increases the tolerable dose of a PKC activator.
116. The method of clause 111, wherein the PKC inhibitor is vitamin E, vitamin E analogs, vitamin E salts, calphostin C, thiazolidinediones, ruboxistaurin or combinations thereof.
117. A method of slowing or reversing the loss of memory and learning comprising the steps of contacting an effective amount of a PKC activator with a protein kinase C (PKC) in a subject identified with memory loss slowing or reversing memory loss.
118. The method of clause 117, wherein the contacting of an effective amount of a PKC activator with ah PKC stimulates cellular or neuronal growth.
119. The method of clause 117, wherein the contacting of an effective amount of a PKC activator with ah PKC stimulates dendritic growth.
120. The method of clause 117, wherein the contacting of an effective amount of a PKC activator with a PKC stimulates dendritic spine formation.
121. The method of clause 117, wherein the contacting of an effective amount of a PKC activator with a PKC stimulates dendritic spine density.
122. A method of stimulating cellular or neuronal growth comprising the steps of contacting a effective amount of a PKC activator with a protein kinase C (PKC) in a subject, thereby stimulating cellular or neuronal growth.
123. The method of clause 122, wherein said subject is identified as having impaired learning or memory.
124. The method of clause 122, wherein the contacting of an effective amount of a PKC activator with a PKC stimulates dendritic growth.
125. The method of clause 122, wherein the contacting of an effective amount of a PKC activator with a PKC stimulates dendritic spine formation.
126. The method of clause 122, wherein the contacting of an effective amount of a PKC activator with a PKC stimulates dendritic spine density.

## Claims

1. A composition for use in stimulating synaptic remodelling in a subject wherein the composition comprises an effective amount of a protein kinase C (PKC) activator which is a macrocyclic lactone and a pharmaceutically acceptable carrier.

2. The composition for use according to claim 1, wherein the macrocyclic lactone is a bryostatin or neristatin.

3. The composition for use according to claim 2, wherein the bryostatin is chosen from bryostatin-1, 2, 3, 4, 5, 6, 7, 8, 9, 10, 11, 12, 13, 14, 15, 16, 17, and 18.

4. The composition for use according to claim 3, wherein the bryostatin is bryostatin-1.

5. The composition for use according to claim 2, wherein the neristatin is neristatin-1.

6. The composition for use according to claim 1, wherein the composition stimulates dendritic spine growth in a subject.

7. The composition for use according to claim 1, wherein the composition stimulates dendritic spine formation in a subject.

8. The composition for use according to claim 1, wherein the composition increases dendritic spine density in a subject.

9. The composition for use according to claim 1, wherein the composition stimulates ELAV translocation to proximal dendrites.
